(19) Europäisches Patentamt / European Patent Office / Office européen des brevets

(11) **EP 1 782 777 A1**

(12) **EUROPEAN PATENT APPLICATION**
published in accordance with Art. 158(3) EPC

(43) Date of publication:
09.05.2007 Bulletin 2007/19

(51) Int Cl.:
*A61F 7/08* (2006.01)    *A61B 19/08* (2006.01)
*C09K 5/16* (2006.01)

(21) Application number: 05765771.0

(22) Date of filing: 14.07.2005

(86) International application number:
PCT/JP2005/013007

(87) International publication number:
WO 2006/006654 (19.01.2006 Gazette 2006/03)

(84) Designated Contracting States:
DE FR GB

(30) Priority: 14.07.2004 JP 2004207835

(71) Applicant: Mycoal Products Corporation
Tochigi-chi, Tochigi 328-0067 (JP)

(72) Inventor: DODO, Toshihiro,
c/o MYCOAL PRODUCTS CORPORATION
Tochigi,
3280 80067 (JP)

(74) Representative: Thun, Clemens et al
Mitscherlich & Partner
Sonnenstrasse 33
80331 München (DE)

(54) **HEATING CLOTH AND PROCESS FOR PRODUCING THE SAME**

(57)     To provide a heat cloth which is thin and flexible; even when as a reaction of an air-permeable heat cloth proceeds, a heat generating composition becomes massive so that flexibility is lowered, or a part of an accommodating bag rides up by shrinkage and curling as caused by heat generation, well keeps the bonding and holding state so that it does not fall off easily; is supple like cloths; is excellent in flexing properties; easily and surely fits to flexible places such as elbows and knees; is able to take warmth; is applicable with good follow-up deformation properties to various places of a human body such as curved parts including shoulders, arms, a neck and feet; and hardly causes an uncomfortable feeling and a process for producing the same.

A heat cloth having a heat generating composition molded body resulting from molding a heat generating composition containing surplus water as a connecting substance accommodated in an accommodating bag, wherein the accommodating bag is constituted of a substrate and a covering material; the covering material covers the heat generating composition molded body as provided on the substrate; the periphery of the heat generating composition molded body is heat sealed to form irregularities; sectional exothermic parts of a convex in which the heat generating composition molded body is accommodated are disposed while holding a sectioned part of a concave as a heat seal part; and the exothermic part is formed of a gathering of the sectional exothermic parts, which is **characterized in that** the substrate is substantially planar and does not contain a pocket, an accommodating division or an accommodating section; that the heat generating composition contains, as essential components, an iron powder, a carbon component, a reaction accelerator and water, does not contain a flocculant aid, a flocculant, an agglomeration aid, a dry binding material, a dry binding agent, a dry binder, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air; that a volume of the heat generating composition molded body is from 0.1 to 30 cm$^3$; that a capacity of the sectional exothermic parts to a ratio of the volume of the heat generating composition molded body is from 0.6 to 1.0; that a maximum height of the sectional exothermic parts is from 0.1 to 10 mm; that a width of the sectioned part as a space between the sectional exothermic parts is from 0.3 to 50 mm; that a minimum bending resistance on the surface orthogonal to at least a thickness of the heat cloth is not more than 100 mm; that a part of the accommodating bag has air permeability; and the surroundings of the accommodating bag are sealed.

EP 1 782 777 A1

# *FIG.2(a)*

# *FIG.2(b)*

**Description**

[Technical Field]

The present invention relates to a heat cloth using a heat generating composition which after producing a heat generation composition molded body, is capable of causing heat generation without moving water in the heat generating composition molded body to a packaging material or a water absorptive sheet, the heat cloth being soft like fabrics and excellent in flexing properties, easily and surely fitting to flexible places such as elbows and knees, being able to take warmth, being applicable with good follow-up deformation properties to various places of a human body such as curved parts including shoulders, arms, a neck and feet and hardly causing an uncomfortable feeling, and to a process for producing the same.

[Background Art]

Heat generating compositions utilizing an oxidation reaction of a metal such as iron have been provided as a powder or granule, or a viscous material or creamy material. Heat cloths utilizing such a heat generating composition are very excellent in view of costs, safety, exothermic temperature, and so on and are already put into practical use as, for example, a so-called chemical body warmer as filled in an air-permeable bag.

In order to obtain a more comfortable feeling for use, there have been proposed various heat generating compositions which design to have shape holding properties and to hold exothermic characteristics while using a thickener, a binding agent, etc. in quest of prevention of deviation of a heat generating composition and fitness to various kinds of shapes.

For example, Patent Document 1 proposes a process for producing a heat generating composition as granulated so as to have an average particle size of 0.5 mm or more and a process for producing a heat generating composition having an improved granular strength by blending from 10 to 20 parts by weight of an adhesive binder component in addition water and granulating.

Also, Patent Document 2 proposes a throwaway body warmer composed of a heat generating composition having shape holding characteristics by adding a powdered thickener such as corn starch and potato starch.

Also, Patent Document 3 proposes a solid heat generating composition as prepared by mixing a binding agent such as CMC in a powdered or granular heat generating composition and compression molding the mixture.

Also, Patent Document 4 proposes a heat generating body as prepared by using a crosslinking agent, etc. and a water absorptive polymer and integrating them under pressure.

Also, Patent Document 5 proposes a heat generating composition in an ink form and/or a creamy form using a thickener so as to have viscosity, a heat generating body and a process for producing the same.

Also, Patent Document 6 proposes a heat generating composition molded body using a binding agent, the surface of which is covered by an air-permeable film material such as CMC, thereby designing to hold the shape.

Also, Patent Document 7 and Patent Document 8 propose a heat generating composition as processed into a viscous material or a creamy material, in which the shape is changed from a conventional rectangle to a foot shape or an elliptical shape so as to adapt to the outline of a body to be warmed.

Also, there is disclosed a heat generating body having a soft structure in which an exothermic part having a heat generating composition sealed between packaging materials at least one surface of which is permeable to air is constituted of plural small exothermic parts as divided by a seal part.

Patent Document 9 and Patent Document 10 each discloses a heat generating body in which a powdered heat generating composition is filled in sectioned divisions and which is made of plural exothermic parts as divided by a seal part.

Also, Patent Document 11, Patent Document 12, Patent Document 13, Patent Document 14, Patent Document 15 and Patent Document 16 each propose a heat generating composition using a flocculant aid and a dry binding agent and a heat generating body in which a heat generating composition exothermic part is sectioned into plural divisions by using a substrate having an accommodating pocket.

However, following spreading in utilization of throwaway body warmers which are aimed to be applied to various places of a human body such shoulders, arms, a neck and feet, even if a heat generating composition is hardened by a thickener, etc., there were encountered problems that in a single packed state, for example, bonding retention is difficult so that the dropping easily occurs and that a strong uncomfortable feeling is caused in wearing. Such problems are promoted due to a lowering of flexibility as caused by blocking following progression of a reaction of heat generating body. There was also encountered a problem that a stretched film which forms an accommodating bag is shrunk and curled due to heat generation so that an end part of a single packaging bag rides up, whereby a body warmer as bonded and held easily peels away and drops due to catch therein.

Furthermore, even if the exothermic part is divided into plural compartments, a heat generating composition as hardened by a flocculant aid, etc. involved a problem that an exothermic performance is deteriorated.

Furthermore, so far, a heat generating body was produced by a filling system or produced by filling a heat generating composition containing a flocculant and a binding agent in a packaging material having accommodating divisions as molding in vacuo an agglomerate or compressed body. Moreover, a heat generating body was produced by previously preparing a filling pocket in a substrate, filling a heat generating composition in the pocket and covering a packaging material thereon, followed by sealing.

Furthermore, in the case of producing a heat generating body having sectioned exothermic parts by using a powdered heat generating composition or a granular heat generating composition as a heat generating composition, according to a method using a filling system, since the powdered heat generating composition or granular heat generating composition is accommodated in an accommodating body in a partially sealed bag form and the whole is then sealed, there was a limit in size of a sectional region in view of the production. That is, according to a method for filling a powdered heat generating composition or a granular heat generating composition while partially sealing, it was mechanically substantially impossible to produce a heat generating body having a plural number of small-sized sectional regions, and additionally, there was caused a problem due to shortage in sealing as caused by incorporation of the heat generating composition into a seal part or the like. In particular, it was substantially impossible to continuously produce one having a partial shape having a size of not more than 20 mm or one having a small shape of not more than 20 mm. Furthermore, according to a method using a rotary magnet system as disclosed in Patent Document 17, not only a complicated operation is necessary, but also its structure is complicated. Accordingly, there were encountered problems that the operation at the time of forming an exothermic layer is troublesome and that a device to be used is complicated and expensive, is liable to cause a fault, takes a long time to do the maintenance and is inconvenient for handling. Therefore, there was a limit in making the size of the exothermic part small.

Furthermore, according to a method using a pocket system, a heat generating composition containing a flocculant and a binding agent is used and a dry powdered mixture of an exothermic component containing a flocculant and a binding agent is filled in a concave pocket as previously prepared in a packaging material directly or after compressing it to form a granule, a pellet, a tablet or a scrub, followed by compression to prepare an exothermic part. Now, in comparison with a heat generating body in which a flocculant and a binding agent are not incorporated, in a heat generating body having plural sectional exothermic parts, the exothermic time is markedly short, especially, in a sectional exothermic part having a narrow region whose shortest length is not more than 15 mm or a sectional exothermic part of a small size, the exothermic duration is markedly short, resulting in a problem in view of practical use. If it is intended to prolong the exothermic duration, there was a problem that it becomes necessary to increase the dimensions of a single sectioned exothermic part so that the heat generating body becomes one having a sectional exothermic part of a large size. Furthermore, even by using a powdered or granular heat generating composition, there was a problem that a concave pocket must be previously provided in a packaging material, thereby bringing a complicated operation. Furthermore, since it is necessary to form a heat generating composition capable of causing heat generation upon contact with air by accommodating it in a pocket, sealing the heat generating composition by a packaging material and then adding water in the heat generating composition within the pocket, a step for achieving this must be provided. Thus, the process became complicated and involved a problem in view of costs.

[Patent Document 1] JP-A-4-293989

[Patent Document 2] JP-A-6-343658

[Patent Document 3] JP-A-59-189183

[Patent Document 4] WO 00/13626

[Patent Document 5] JP-A-9-75388

[Patent Document 6] JP-A-60-101448

[Patent Document 7] JP-A-9-276317

[Patent Document 8] JP-A-11-299817

[Patent Document 9] JP-UM-A-1-110718

[Patent Document 10] JP-UM-A-6-26829

[Patent Document 11] JP-A-2000-288008

[Patent Document 12] JP-T-11-507593

[Patent Document 13] JP-T-11-508314

[Patent Document 14] JP-T-11-508786

[Patent Document 15] JP-T-11-512954

[Patent Document 16] JP-T-2002-514104

[Patent Document 17] JP-A-7-124193

[Disclosure of the Invention]

[Problems that the Invention is to Solve]

An object of the invention is to solve the foregoing problems and to provide a heat cloth which is thin and flexible; even when as a reaction of an air-permeable heat cloth proceeds, a heat generating composition becomes massive so that flexibility is lowered, or a part of an accommodating bag rides up by shrinkage and curling as caused by heat generation, well keeps the bonding and holding state so that it does not fall off easily; is supple like cloths; is excellent in flexing properties; easily and surely fits to flexible places such as elbows and knees; is able to take warmth; is applicable with good follow-up deformation properties to various places of a human body such as curved parts including shoulders, arms, a neck and feet; and hardly causes an uncomfortable feeling and a process for producing the same.

[Means for Solving the Problems]

An object of the invention is to solve the foregoing problems and to provide a heat cloth which is thin and flexible; even when as a reaction of an air-permeable heat cloth proceeds, a heat generating composition becomes massive so that flexibility is lowered, or a part of an accommodating bag rides up by shrinkage and curling as caused by heat generation, well keeps the bonding and holding state so that it does not fall off easily; is supple like cloths; is excellent in flexing properties; easily and surely fits to flexible places such as elbows and knees; is able to take warmth; is applicable with good follow-up deformation properties to various places of a human body such as curved parts including shoulders, arms, a neck and feet; and hardly causes an uncomfortable feeling and a process for producing the same. Specifically, as set forth in claim 1, a heat cloth of the invention is a heat cloth having a heat generating composition molded body resulting from molding a heat generating composition containing surplus water as a connecting substance accommodated in an accommodating bag, wherein the accommodating bag is constituted of a substrate and a covering material; the covering material covers the heat generating composition molded body as provided on the substrate; the periphery of the heat generating composition molded body is heat sealed to form irregularities; sectional exothermic parts of a convex in which the heat generating composition molded body is accommodated are disposed while holding a sectioned part of a concave as a heat seal part; and the exothermic part is formed of a gathering of the sectional exothermic parts, which is characterized in that:

1) the substrate is substantially planar and does not contain a pocket, an accommodating division or an accommodating section,
2) the heat generating composition contains, as essential components, an iron powder, a carbon component, a reaction accelerator and water, does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air,
3) a volume of the heat generating composition molded body is from 0.1 to 30 cm$^3$,
4) a ratio of the capacity of the sectional exothermic parts to the volume of the heat generating composition molded body is from 0.6 to 1.0,
5) a maximum height of the sectional exothermic parts is from 0.1 to 10 mm,
6) a width of the sectioned part as a space between the sectional exothermic parts is from 0.3 to 50 mm,
7) a minimum bending resistance on the surface orthogonal to at least a thickness of the heat cloth is not more than 100 mm,
8) a part of the accommodating bag has air permeability, and
9) the surroundings of the accommodating bag are sealed.

Also, a heat cloth as set forth in claim 2 is characterized in that in the heat cloth as set forth in claim 1, the heat generating composition contains a component resulting from a contact treatment of a mixture containing at least an iron powder, a carbon component, a reaction accelerator and water as essential components with an oxidizing gas.
Also, a heat cloth as set forth in claim 3 is characterized in that in the heat cloth as set forth in claim 1, the iron powder comprising particles, a surface of each of which is at least parially covered with an iron oxide film, the iron oxide film has a thickness of 3 nm or more, and the iron powder at least contains from 20 to 100 % by weight of an active iron powder particles having a region of an oxygen-free iron component in at least one region selected from a central part region of the iron powder particles and a region beneath the iron oxide film.
Also, a heat cloth as set forth in claim 4 is characterized in that in the heat cloth as set forth in claim 1, the iron powder is covered on at least a part of the surface thereof by a wustite film and contains from 20 to 100 % by weight of an active iron powder having an amount of wustite of from 2 to 50 % by weight in terms of an X-ray peak intensity ratio to iron.

Also, a heat cloth as set forth in claim 5 is characterized in that in the heat cloth as set forth in claim 1, the heat generating composition molded body is compressed.

Also, a heat cloth as set forth in claim 6 is characterized in that in the heat cloth as set forth in claim 1, the heat seal part is formed by heat sealing after temporary adhesion by an adhesive layer, and an adhesive component which constitutes the adhesive layer and a heat seal material component which constitutes the heat seal part are copresent in at least a part of the heat seal part.

Also, a heat cloth as set forth in claim 7 is characterized in that in the heat cloth as set forth in claim 1, after heat sealing, at least a part of the heat generating composition molded body is moved to a temporary adhering part which is not heat sealed, thereby deadhering the temporary adhering part which is not heat sealed.

Also, a heat cloth as set forth in claim 8 is characterized in that in the heat cloth as set forth in claim 1, at least a part of the air-permeable surface of the exothermic part in which the sectional exothermic parts are continuously provided is covered by an air permeability adjusting material.

Also, a heat cloth as set forth in claim 9 is characterized in that in the heat cloth as set forth in claim 1, the sectioned part is provided with a perforation.

Also, a heat cloth as set forth in claim 10 is characterized in that in the heat cloth as set forth in claim 1, at least a part of the sectioned part has an irregular pattern.

Also, a heat cloth as set forth in claim 11 is characterized in that in the heat cloth as set forth in claim 1, the accommodating bag has a fixing measure on at least a part of the exposed surface thereof.

Also, a heat cloth as set forth in claim 12 is characterized in that in the heat cloth as set forth in claim 11, the fixing measure is an adhesive layer and optionally is provided with a separator.

Also, a heat cloth as set forth in claim 13 is characterized in that in the heat cloth as set forth in claim 12, the adhesive layer is a hydrophilic adhesive layer, and a packaging material between the hydrophilic adhesive layer and the heat generating composition molded body has a moisture permeability of not more than 2 $g/m^2/24$ hr.

Also, a heat cloth as set forth in claim 14 is characterized in that in the heat cloth as set forth in claim 12, the adhesive layer has air permeability.

Also, a heat cloth as set forth in claim 15 is characterized in that in the heat cloth as set forth in claim 12, the fixing measure is a sheet-like material in which a non-stretchable portion and a stretchable portion are integrally formed in a sheet-like form, and the heat cloth is provided in the non-stretchable portion of the sheet-like material.

Also, a heat cloth as set forth in claim 16 is characterized in that in the heat cloth as set forth in claim 1, the minimum bending resistance on the surface orthogonal to the thickness direction of the heat cloth is not more than 100 mm.

Also, a heat cloth as set forth in claim 17 is characterized in that in the heat cloth as set forth in claim 1, the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

Also, as set forth in claim 18, a process for producing a heat cloth of the invention is a process for producing a heat cloth having a heat generating composition molded body accommodated in an air-permeable accommodating bag, which is characterized in that:

1) the accommodating bag is constituted of a substrate and a covering material; the heat generating composition molded body is formed by molding a heat generating composition containing surplus water as a connecting substance; the covering material covers the heat generating composition molded body as provided on the substrate; the periphery of the heat generating composition molded body is heat sealed to form irregularities; sectional exothermic parts of a convex in which the heat generating composition molded body is accommodated are disposed while leaving a sectioned part of a concave as a heat seal part; and the exothermic part is formed of a gathering of the sectional exothermic parts,

2) the substrate is substantially planar and does not contain a pocket, an accommodating division or an accommodating section,

3) the heat generating composition contains, as essential components, an iron powder, a carbon component, a reaction accelerator and water, with an amount of water contained in the heat generating composition being from 1 to 60 % by weight, does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air,

4) a volume of the heat generating composition molded body is from 0.1 to 30 $cm^3$, and a ratio of the capacity of the sectional exothermic parts to the volume of the heat generating composition molded body is from 0.6 to 1.0,

5) a maximum height of the sectional exothermic parts is from 0.1 to 10 mm,

6) a width of the sectioned part as a space between the sectional exothermic parts is from 0.3 to 50 mm,

7) a minimum bending resistance on the surface orthogonal to at least a thickness of the heat cloth is not more than 100 mm,

8) a part of the accommodating bag has air permeability, and

9) the surroundings of the accommodating bag are sealed.

Also, a process for producing a heat cloth as set forth in claim 19 is characterized in that in the process for producing a heat cloth as set forth in claim 18, the substrate and the covering material each have a heat seal layer; an adhesive layer made of an adhesive is provided on at least one of the heat seal layers; and the substrate and the covering material are temporarily adhered via the sticky layer in the periphery of the heat generating composition molded body and then heat sealed.

Also, a process for producing a heat cloth as set forth in claim 20 is characterized in that in the process for producing a heat cloth as set forth in claim 19, heat sealing is carried out in a width narrower than that of the temporary adhering seal part, and thereafter, the heat generating composition is moved into a region which is not heat sealed within the temporary adhering seal part, thereby achieving deadhesion.

Also, in the heat cloth, it is preferable that a thermal buffer sheet is provided in the central part of the adhesive layer.

Also, in the process for producing a heat cloth, it is preferable that force-through molding or cast molding is employed for molding the heat generating composition.

Also, in the process for producing a heat cloth, it is characterized that the heat generating composition is compressed within a die.

[Advantages of the Invention]

As is clear from the foregoing description, the following advantages are brought.

1. Since the heat cloth of the invention has an exothermic part resulting from sealing a heat generating composition molded boy using a heat generating composition by a substrate and a covering material, which has a thin and fine form and does not deviate the heat generating composition, it is soft like fabrics and excellent in flexing properties, easily and surely fits to flexible places such as elbows and knees, is able to take warmth, is applicable with good follow-up deformation properties to various places of a human body such as curved parts including shoulders, arms, a neck and feet and hardly causes an uncomfortable feeling. Furthermore, while making the best use of the thin and small-sized shape, the heat cloth of the invention can be applied to hemorrhagic goods or sanitary napkins.

2. Since the heat cloth of the invention does not contain a flocculant aid, a flocculant, an agglomeration aid, a dry binding material, a dry binding agent, a dry binder, an adhesive binder, a thickener and an excipient and uses surplus water having a water mobility value of from 0.01 to 20 to impart moldability, it is not required to provide an accommodating pocket in the substrate. Furthermore, the heat cloth of the invention can be produced by using a substantially planar substrate and is excellent in exothermic characteristics so that a sufficiently effective exothermic time can be taken even in the case of an ultra-thin heat cloth.

3. By combining a temporary adhesion measure, the heat cloth can be produced at a higher speed.

4. The exothermic part is made of sectional exothermic parts in a stripe form, and a minimum bending resistance in one direction on the surface orthogonal to the thickness direction of the heat cloth is not more than 60 mm. Thus, the heat cloth of the invention is excellent in adhesion to curved surfaces of the body.

5. The sectional exothermic parts constitute a high part, and the sectioned part constitutes a bottom part; the high part and the low part are alternately present; by combining an air permeability material, a firm space can be held; and the space is present along the heat generating composition, whereby the whole of the heat generating composition can start an exothermic reaction simultaneously with ventilation. Thus, the heat cloth could be warmed from the periphery to the central part thereof without causing uneven temperature, and heat insulation of the heat generating composition, uniformity of heat generation and prolongation of an exothermic time could be realized.

In the light of the above, the invention is able to provide a heat cloth only by laminating a heat generating composition molded body resulting from molding a heat generating composition having surplus water having a water mobility value of from 0.01 to 20 on a substrate, covering a covering material thereon and sealing at least the periphery of the heat generating composition molded body, does not require the addition of water after accommodating it in a packaging material of the substrate or covering material so that the process is remarkably simplified. Thus, the invention has superiority in view of costs. That is, the invention is concerned with a heat cloth using a heat generating composition molded body resulting from molding a moldable heat generating composition containing surplus water as a connecting substance, wherein the heat generating composition does not contain a flocculant aid, a dry binding agent and a flocculant but contains an appropriate amount of surplus water as expressed in terms of a water mobility value as a connecting substance. When an appropriate amount of the surplus water is contained in the heat generating composition, it is assumed that the surplus water causes hydration with a hydrophilic group in the components of the composition by a bipolar mutual action or hydrogen binding and is present in the surroundings of a hydrophobic group while having high structural properties. In this way, it is assumed that a sand dumpling state is formed, whereby moldability of the heat generating composition is revealed. This surplus water is connecting water as a connecting substance for some meaning. Besides, there is water in a state called as free water. It is thought that when the surplus water increases, the structure is softened, and free water is observed. Furthermore, in order that the iron powder causes an oxidation reaction, the existing amount of water and the feed amount of oxygen onto the surface of the iron powder become a control factor. It is said that the water is not sufficient for about an adsorbing water film (up to 100 angstroms), and an oxidation rate is small. When the adsorbing film is about 1 $\mu$m, not only the amount of water is sufficient, but also the feed of oxygen onto the surface of the iron powder is easy because the thickness of the water film is thin, thereby exhibiting a large oxidation rate. When the film becomes thicker and the adsorbing film becomes thick exceeding 1 $\mu$m, the feed amount of oxygen is reduced. As a result of obtaining knowledge that one expressing an optimal amount of water at which moldability and oxidation rate in fixed or higher levels are exhibited is a water mobility value and is from 0.01 to 20, the invention has been accomplished. That is, by using an appropriate amount of surplus water, the particles of the respective components are secured by a surface tension of the water. Thus, moldability is revealed in the heat generating composition, and the water does not function as a barrier layer. Accordingly, the heat generating composition to be used in the invention comes into contact with air to cause heat generation. In addition, by using a heat generating composition using an active carbon powder or an active heat generating composition, it is possible to use a heat generating composition having markedly excellent exothermic rising properties and improved moldability. Furthermore, the heat cloth has two or more sectional exothermic parts as produced by laminating the heat generating composition molded body resulting from molding the moldable heat generating composition on a substantially planar substrate, further covering the covering material on the heat generating composition molded body and then heat sealing. It is possible to provide a heat cloth which is able to cause heat generation without moving water in the heat generating composition molded body as produced by a molding and lamination system to the packaging material or water absorptive sheet, has flexibility by itself, is excellent in wearing on each place of a human body and places as required to have flexibility, such as materials having a curved surface and is excellent in feeling for use and a process for producing the same. Furthermore, among the substrate, covering material and heat generating composition molded body, by temporarily adhering at least the covering material and the heat generating composition molded body via a sticky layer and then heat sealing the periphery of the heat generating molded body and the surroundings of the heat cloth, reliability of the heat sealing is improved. Thus, it is possible to design to achieve high-speed production of a heat cloth and make the heat seal width small.

[Best Modes for Carrying Out the Invention]

The heat cloth of the invention is a a heat cloth having a heat generating composition molded body resulting from molding a heat generating composition containing surplus water as a connecting substance accommodated in an accommodating bag, wherein the accommodating bag is constituted of a substrate and a covering material; the covering material covers the heat generating composition molded body as provided on the substrate; the periphery of the heat generating composition molded body is heat sealed to form irregularities; sectional exothermic parts of a convex in which the heat generating composition molded body is accommodated are disposed while holding a sectioned part of a concave as a heat seal part; and the exothermic part is formed of a gathering of the sectional exothermic parts, which is characterized in that:

> 1) the substrate is substantially planar and does not contain a pocket, an accommodating division or an accommodating section,
> 2) the heat generating composition contains, as essential components, an iron powder, a carbon component, a reaction accelerator and water, does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air,
> 3) a volume of the heat generating composition molded body is from 0.1 to 30 $cm^3$,
> 4) a capacity of the sectional exothermic parts to a ratio of the volume of the heat generating composition molded body is from 0.6 to 1.0,
> 5) a maximum height of the sectional exothermic parts is from 0.1 to 10 mm,
> 6) a width of the sectioned part as a space between the sectional exothermic parts is from 0.3 to 50 mm,
> 7) a minimum bending resistance on the surface orthogonal to at least a thickness of the heat cloth is not more than 100 mm,
> 8) a part of the accommodating bag has air permeability, and

9) the surroundings of the accommodating bag are sealed.

In addition, in the heat cloth of the invention, for the purposes of achieving high-speed heat sealing, making the heat seal width thin and achieving sure heat sealing, there is also employed heat sealing after temporary adhesion in which the substrate and the covering material are temporarily adhered via a sticky layer, followed by heat sealing. That is, the substrate and the covering material of the air-permeable accommodating bag each have a heat seal layer; a heat seal part is formed by the heat seal layer; the heat seal part is formed by heat sealing after temporary adhesion with an adhesive layer to form a temporary adhering seal; and an adhesive component which constitutes the adhesive layer and a heat seal component which constitutes the heat seal layer are copresent in the heat seal part.

By making the heat seal part width which sections the exothermic part thin, a space between the sectional exothermic parts becomes narrow, a mutual heat insulation effect is kept, and the exothermic part can be sectioned without causing a lowering of exothermic characteristics such as a decrease in exothermic time to be caused due to sectioning of the exothermic part, whereby a heat cloth having flexibility and excellent exothermic characteristics can be obtained.

In the sectional exothermic part or the heat generating composition molded body of the invention, its maximum width is usually from 0.5 to 60 mm, preferably from 0.5 to 50 mm, more preferably from 1 to 50 mm, further preferably from 3 to 50 mm, still further preferably 3 to 30 mm, even further preferably from 5 to 20 mm, even still further preferably from 5 to 15 mm, and most preferably from 5 to 10 mm. Furthermore, its maximum height is usually from 0.1 to 30 mm, preferably from 0.1 to 10 mm, more preferably from 0.3 to 10 mm, further preferably from 1 to 10 mm, and still further preferably from 2 to 10 mm. Moreover, its longest length is usually from 5 to 300 mm, preferably from 5 to 200 mm, more preferably from 5 to 100 mm, further preferably from 20 to 150 mm, and still further preferably from 30 to 100 mm.
A capacity of the sectional exothermic part or a volume of the heat generating composition molded body is usually from 0.015 to 500 cm$^3$, preferably from 0.04 to 30 cm$^3$, more preferably from 0.1 to 30 cm$^3$, further preferably from 1 to 30 cm$^3$, and still further preferably from 3 to 20 cm$^3$.

In the sectional exothermic part, when the sectional exothermic part which is an accommodating region of the heat generating composition is filled with the heat generating composition molded body, a volume ratio of the volume of the heat generating composition molded body which is an occupying region of the heat generating composition molded body to the capacity of the sectional exothermic part which is an accommodating region of the heat generating composition is usually from 0.6 to 1, preferably from 0.7 to 1, more preferably from 0.8 to 1, and further preferably from 0.9 to 1.0.

Furthermore, a width of the sectioned part which is a space between the sectional exothermic parts is not limited so far as sectioning can be achieved. It is usually from 0.1 to 50 mm, preferably from 0.3 to 50 mm, more preferably from 0.3 to 50 mm, further preferably from 0.3 to 40 mm, still further preferably from 0.5 to 30 mm, even further preferably from 1.0 to 20 mm, and even still further preferably from 3 to 10 mm.
Incidentally, the heat generating composition molded body or the sectional exothermic part may have any shape. The shape may be a planar shape, and examples thereof include a circular shape, an elliptical shape, a polygonal shape, a star shape, and a flower shape. Also, the shape may be a three-dimensional shape, and examples thereof include a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape. Furthermore, in these shapes, the corner may be rounded, thereby processing the corner in a curvilinear or curved state, or the central part may be provided with a concave.
Furthermore, the "volume of the heat generating composition molded body of the invention" as referred to herein means a volume of the heat generating composition molded body or compressed heat generating composition molded body.
Furthermore, the "capacity of the sectional exothermic part" as referred to herein means an internal capacity of the sectional exothermic part having a heat generating composition molded body accommodated therein.

In the sectional exothermic part, the accommodating bag, the outer bag (accommodating bag of the heat generating body), and the like, packaging materials or the like constituting the same are sealed in the sectioned part as a seal part or its periphery or surroundings. Though heat seal is usually employed, other seal method can be employed depending upon the utility. As one example, sealing is carried out in a point-like (intermittent) manner or entirely by compression seal (adhesive seal), warm compression seal (adhesive seal), bonding seal, heat bonding seal, heat melt seal (heat seal), etc. by means of pressurizing, warming, heating or a combination thereof via an adhesive layer and/or a bonding agent layer and/or a heat seal layer. Selection of any one or a combination of these methods may be made depending upon the desire. In this way, it is possible to seal and form a sectional exothermic part, an inner bag (accommodating bag), an outer bag, etc. Sewing processing can also be employed as one of seal means.

A heat cloth in which a number of sectional exothermic parts are continuously provided and a perforation is provided to a degree such that cutting by hand is possible in the sectioned part can be cut into an appropriate size at the time of use on the basis of the purpose for use adaptive with a place for application of a human body, or the like and applied. In that case, the size of the heat cloth and the size and number of the sectional exothermic parts may be properly set up. There are no limitations regarding such size and number. Furthermore, the sectioned part can be formed in arbitrary directions such as a length or width direction, length and width directions, and an oblique direction.

Furthermore, at least one surface of a heat generating body having two or more sectional exothermic parts connected to each other may be covered by a packaging material. A heat generating body may be formed by connecting two or more sectional exothermic parts to each other, or a heat generating body may be formed by covering at least one surface of the connected sectioned exothermic parts. As the packaging material, the raw material which is used in the substrate, the covering material or the underlay material can be used.

For example, in the case of using a heat seal layer-containing film as the packaging material to produce a heat generating body, a heat generating body may be produced by using a perforated heat sealable plastic film as an air-permeable packaging material and sticking a non-woven fabric in the air-permeable side thereof via an air-permeable adhesive layer, thereby keeping warmth at the time of use or preventing leakage of collapsed pieces of the heat generating composition, or a heat generating body for thermal muffler may be formed by wrapping the both surfaces by a non-woven fabric, etc.

At least a part of the surface of the heat generating composition molded body may be covered by an air-permeable adhesive layer made of a net-work hot melt based adhesive, etc., or an underlay material such as a non-woven fabric may be provided between the air-permeable adhesive layer and the covering material.

Furthermore, at least one member of the heat generating composition molded body, the substrate, the covering material, the air-permeable adhesive layer, and the underlay material may be entirely or partly subjected to a pressurizing treatment or provided with irregularities. In this may, the transfer of the heat generating composition molded body between the substrate and the covering material may be prevented.

That is, a molded body prepared by appropriately compressing the heat generating composition molded body of the invention under pressure is markedly improved in moldability. For example, even when a perforated film which is difficult with respect to the pressure adjustment is used as a raw material of the air-permeable part in place of the porous film, in the case where an inner pressure of the accommodating bag becomes equal to or more than the outer pressure, shape collapse hardly occurs so that the use of a perforated film is possible. Accordingly, not only the range for selecting an air-permeable raw material is widened so that the costs can be lowered, but also a body to be warmed can be uniformly warmed at an appropriate temperature over a long period of time.

Incidentally, in the case where the heat generating composition molded body of the invention is compressed, a rate of compression of the heat generating composition compressed body is preferably from 50 to 99.5 %, more preferably from 60 to 99.5 %, further preferably from 60 to 95 %, still further preferably from 65 to 95 %, and even further preferably from 70 to 90 % in thickness with respect to the thickness before the compression. Furthermore, with respect to the thickness before the compression, a thickness of a die at the time of die molding can be employed.

In the exothermic part, for the purpose of containing a magnetic substance in at least a part thereof or one sectional exothermic part to improve the blood circulation or stiff shoulders due to a magnetic effect, it is also possible to accommodate therein a magnetic substance such as a magnet.

The shape of the heat cloth may be any shape and can be selected from the group consisting of a rectangular shape, a circular shape, an elliptical shape, a polygonal shape, a broad bean-like shape, an eye mask-like shape, a cocoon-like shape, a gourd-like shape, a rectangular shape with rounded corners, a square shape with rounded corners, an egg-like shape, a boomerang-like shape, a comma-shaped bead-like shape, a wing-like shape, a nose-like shape, a star-like shape, and a foot-like shape.

The cross-sectional shape of the sectioned part which is the seal surface may be formed irregularly, thereby providing a pattern.

The cross-sectional shape of the seal surface may be a planar and plain surface. However, for the purposes of not only making fashionableness rich and imparting visible pleasant but also making slipperiness small in forwarding the heat cloth, it is preferred to form irregularities to provide a pattern.

It is not always required that this pattern is provided over the whole of the seal surface. A pattern may be provided only in the side of the sectional exothermic part, with the remainder being not provided with a pattern. Inversely, no pattern may be provided in the side of the sectional exothermic part, with the remainder being provided with a pattern.

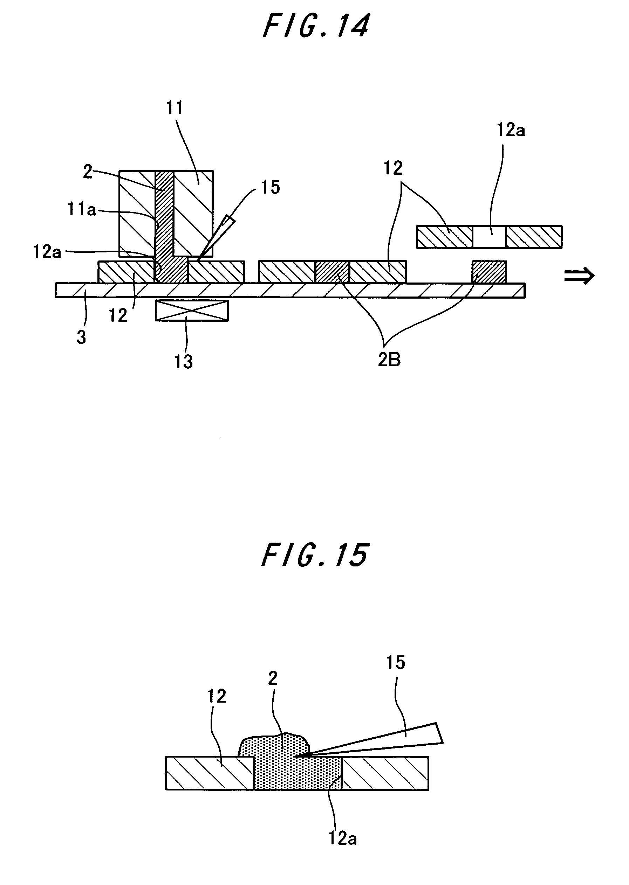

The pattern on the seal surface is not particularly limited so far as the cross-sectional shape is irregular. Examples thereof include an orthogonal lattice shape, a parallel vertical linear shape, a parallel horizontal linear shape, a zigzag shape, an oblique lattice shape, a broken oblique linear shape, an oblique checkerwork shape, and a scattered point shape. Figs. 14 (a) to 14 (q) show examples of a specific combination of a pattern and a plain surface. For example, no pattern may be provided in the side of the sectional exothermic part, with the central part being patterned. Inversely, the side of the sectional exothermic part is patterned, with the remainder as a central part being not provided with a pattern. The pattern can be arbitrarily chosen. Furthermore, a pattern in the surroundings as the circumferential part of the heat cloth is the same.

A method for providing a pattern on the seal surface is not limited. Examples thereof include a method in which a pattern having an irregular cross-sectional shape is provided in a seal part of a seal bar or a seal roll which is a seal mold and sealing is carried out using the seal mold.

Furthermore, the invention is also applicable as a thermal sheet for operation capable of keep the temperature by warming a sheet to be covered over the body of a human being during the operation. In this way, it is possible to prevent the body from the cold during the operation. Furthermore, by applying heat to a specific region of the body of a human being who suffers from a pain, it is possible to remedy an acute or chronic knee including a muscle, a bone structure and a pain, or the like.

In the case of application to the body during the operation, though there are no particular limitations, it is preferable that heating and heat insulation are preferably carried out for from 0.5 to 24 hours, more preferably from 3 to 24 hours, further preferably from 6 to 24 hours, and still further preferably from 8 to 16 hours and that the skin temperature is kept at from about 32 °C to about 40 °C, preferably from about 32 °C to about 39 °C, more preferably from about 32 °C to about 37 °C, and further preferably from about 32 °C to about 36 °C.

Incidentally, though the size of the sheet is not limited, it is preferably longitudinal, lightweight and thin.

Furthermore, by combining the substrate, the covering material, the heat generating composition and the adhesive layer to adjust a minimum bending resistance in one direction on the surface orthogonal to the thickness direction of the heat cloth at not more than 100 mm, it is possible to prepare a heat cloth having enhanced adhesion to a body to be warmed such as the body and having an excellent feeling for use.

Furthermore, at least one or a part of the substrate, the covering material, the air permeability adjusting material, the adhesive layer, and the separator, each of which constitutes the heat cloth, may be provided with at least one member of characters, designs, symbols, numerals, patterns, photographs, pictures, and colors.

The substrate, the covering material, the air permeability adjusting material, and the adhesive layer, each of which constitutes the heat cloth, may be transparent, opaque, colored, or colorless. Furthermore, a layer constituting at least one layer of the layers constituting the respective materials and layers may be colored to a color different from those of other layers.

The heat generating body is accommodated in an air-tight air-impermeable accommodating bag, stored and transported. Examples thereof include a heat generating body prepared by interposing a produced heat generating body between two sheets of an air-impermeable film or sheet, punching the two sheets of film or sheet into a size larger than that of the heat generating body at the same time with or after this interposition, and sealing the two sheets of film or sheet in the surroundings exceeding the size of the heat generating body at the same time with or after this punching. The outer bag is not limited so far as it is air-impermeable and may be made of a laminate. Usually, an outer bag prepared from an air-impermeable raw material is used.

The accommodating bag of the invention is made of a substrate and a covering material, and in addition, an underlay material may be provided between the substrate and the covering material. Each of the substrate and the covering material is substantially planar and does not contain a pocket, an accommodating division or an accommodating section; the covering material covers the heat generating composition provided on the substrate; the sectional exothermic part which is constituted by heat sealing the periphery of the heat generating composition is made of two or more plural sectional exothermic parts; the respective exothermic parts are disposed at intervals by the sectioned part which is a heat seal part; and the exothermic part is formed of a gathering of the foregoing sectional exothermic parts. Here, in the invention, the substrate and the covering material are not distinguished from each other depending upon a raw material constitution; but a raw material on which the heat generating composition molded body is laminated is defined as a substrate, and a raw material which is then covered on the substrate or heat generating composition molded body is defined as a covering material.

A raw material of the substrate or covering material is not limited so far as it functions as an accommodating bag of the heat generating composition. Usually, raw materials which are used in chemical body warmers or heat generating bodies can be used. Examples of the raw material include air-impermeable raw materials, air-permeable raw materials, water absorptive raw materials, non-water absorptive raw materials, non-extensible raw materials, extensible raw materials, stretchable raw materials, non-stretchable raw materials, foamed raw materials, non-foamed raw materials, non-heat sealable raw materials, and heat sealable raw materials. The raw material can be properly used depending upon a desired utility in a desired form such as films, sheets, non-woven fabrics, woven fabrics, and composites thereof. In general, the substrate is made of an air-impermeable film or sheet, and the covering material is made of an air-permeable film or sheet or non-woven fabric, and vice versa. The both may be air-permeable. As the underlay material, an air-permeable underlay material and an air-impermeable underlay material may be used for different purposes.

The packaging material of the accommodating bag may be of a single-layered structure or multilayered structure, and its structure is not limited. Furthermore, though the packaging material is composed of at least a substrate and a covering material, a packaging material for laminating the heat generating composition molded body is the substrate, and a packaging material for covering on the heat generating composition molded body is the covering material regardless of whether the packaging material is air-permeable or air-impermeable. An embodiment of a multilayered structure in which an air-impermeable packaging material is the substrate and an air-permeable packaging material is the covering material will be hereunder described as one example.

That is, in this embodiment, the substrate is made of layer A/layer B, layer A/layer B/layer C, or layer A/layer B/layer C/layer D; and the covering material is made of layer F/layer G, layer E/layer F/layer G, or layer F/layer H/layer G. Examples of the layer A include thermoplastic resin films (for example, polyethylene), heat seal layers (for example, polyethylene and EVA), and water absorptive papers; examples of the layer B include non-woven fabrics of a thermoplastic resin (for example, nylons), non-water absorptive papers, water absorptive papers, thermoplastic resin films (for example, polyethylene films, polypropylene films, polyester films, and polyamide (for example, nylons) films), wicks (for example, non-water absorptive papers and water absorptive papers); examples of the layer C include adhesive layers, non-water absorptive papers, water absorptive papers, thermoplastic resin films (for example, polyethylene), non-slip layers, and non-woven fabrics of a thermoplastic resin (for example, polyesters and nylons); examples of the layer D include separators, thermoplastic resin films (for example, polyethylene), and non-woven fabrics; examples of the layer E include heat seal layers; examples of the layer F include porous films or perforated films made of a thermoplastic resin (for example, polyethylene), films made of a thermoplastic resin (for example, polyethylene), non-water absorptive papers, and water absorptive papers; examples of the layer G include non-woven fabrics of a thermoplastic resin (for example, polyesters and nylons) ; and examples of the layer H include non-water absorptive papers and water absorptive papers. Examples of the substrate or covering material include heat seal layer made of polyethylene obtained by using a metallocene catalyst/polypropylene film, polyethylene-made heat seal layer/polypropylene film, EVA-made heat seal layer/polypropylene film, EVA-made heat seal layer/polypropylene film/adhesive layer/separator, EVA-made heat seal layer/polyethylene film/nylon non-woven fabric, non-woven fabric/porous film, heat seal layer made of polyethylene obtained by using a metallocene catalyst/polyethylene film/nylon non-woven fabric, heat seal layer made of polyethylene obtained by using a metallocene catalyst/polypropylene film/polypropylene non-woven fabric, non-woven fabric/(paper and/or perforated (provided by a needle or laser) film)/porous film, non-woven fabric/(paper and/or porous film) /perforated (provided by a needle or laser) film, and non-woven fabric/(paper and/or porous film)/non-woven fabric. A method for laminating the respective layers is not limited. The respective layers may be directly laminated; the respective layers may be laminated via an air-permeable adhesive layer or a laminating agent layer; and the respective layers may be laminated by hot melt extrusion or the like. Furthermore, in the invention, it is to be noted that polyethylene produced by using a metallocene catalyst is also included in the polyethylene.

For example, in the case of laminating the foregoing raw material such as non-woven fabrics and porous films via an air-permeable sticky layer, examples of a method for forming the air-permeable sticky layer include a method in which a sticky substance is fibrillated by an appropriate system such as a curtain spray system, a melt blow system or a slot spray system for blowing and spreading a sticky substance via hot air under heat melting and spread and accumulated on an appropriate supporting substrate made of a porous film, an air-permeable substrate, a separator, etc., thereby forming a porous sticky layer.

A thickness of each of the substrate, the covering material, the underlay material, and the raw material constituting the same varies depending upon the utility and is not limited. The thickness is usually from 5 to 5,000 $\mu$m, preferably from 10 to 500 $\mu$m, and more preferably from 20 to 250 $\mu$m.

The air-impermeable raw material is not limited so far as it is air-impermeable. Examples thereof include films, sheets or coatings made of a polymer (for example, polyethylene, polypropylene, nylons, polyacrylates, polyesters, polyvinyl alcohols, and ethylene-vinyl acetate copolymers) and laminates thereof with a metal (including a semiconductor) compound (for example, silicon oxide) or composite raw materials using the same.

Of the foregoing air-impermeable raw materials, examples of a film having high air impermeability include films provided with a single layer or multiple layers of a thin film having a metal including a semiconductor or a compound thereof provided on an air-impermeable raw material film. Examples of the metal including a semiconductor include silicon, aluminum, and alloys or mixtures containing such a metal. Examples of the metal (including a semiconductor) compound include oxides, nitrides and oxynitrides of the foregoing metals or alloys or mixtures. Examples of the layer include silicon oxide layers, aluminum oxide layers, and silicon oxynitride layers; layers obtained by laminating an arbitrary layer of these layers on a polyester-made film; and layers obtained by further laminating a stretched polyolefin film (for example, a biaxially stretched polypropylene film) thereon.

The air-permeable raw material is not limited so far as it is air-permeable. Examples thereof include air-permeable films (for example, porous films and perforated films); materials having air permeability by themselves (for example, papers and non-woven fabrics); materials prepared by laminating at least one of papers and air-permeable films and non-woven fabrics so as to have air permeability; materials prepared by providing an air-impermeable packaging material comprising a non-woven fabric having a polyethylene film laminated thereon with fine pores by using a needle, etc. so as to have air permeability; non-woven fabric whose air permeability is controlled by laminating a fiber and heat bonding under pressure; porous films; and materials prepared by sticking a non-woven fabric onto a porous film. The "perforated film" as referred to herein is a film prepared by providing an air-impermeable film (for example, polyethylene films) with fine pores by using a needle so as to have air permeability.

The air permeability is not limited so far as the heat generation can be kept. In the case of use in usual heat generation, the air permeability is usually from 50 to 10, 000 g/m$^2$/24 hr, preferably from 70 to 5,000 g/m$^2$/24 hr, more preferably from 100 to 2, 000 g/m$^2$/24 hr, and further preferably from 100 to 700 g/m$^2$/24 hr in terms of moisture permeability by the Lyssy method.

When the moisture permeability is less 50 g/m$^2$/24 hr, the heat value is small and a sufficient thermal effect is not obtained, and therefore, such is not preferable. On the other hand, when it exceeds 10,000 g/m$^2$/24 hr, the exothermic temperature is high so that a problem in safety may possibly be generated, and therefore, such is not preferable. However, there is no limitation even when the moisture permeability exceeds 10,000 g/m$^2$/24 hr depending upon the utility, or even in the use at a moisture permeability closed to the open system, according to circumstances.

The stretchable packaging material is not particularly limited so far as it is stretchable. That is, it is only required that the stretchable packaging material is stretchable as a whole. The stretchable packaging material may be formed of a single material or a composite material of stretchable substrates or a combination of a stretchable substrate and a non-stretchable substrate.

Examples of the stretchable packaging material include single materials (for example, natural rubbers, regenerated rubbers, synthetic rubbers, elastomers, and stretchable shape memory polymers) and mixtures thereof, mixed materials or blended materials of such a stretchable raw material and a non-stretchable raw material or fabrics constituted of a combination of these materials, films, yarns, strands, ribbons, tapes, and stretchable films with a scrim structure.

The porous film is not limited and can be properly selected among porous films obtained by stretching a film made of a polyolefin based resin (for example, polyethylene, linear low density polyethylene, and polypropylene) or a fluorine based resin (for example, polytetrafluoroethylene) and a filler.

The non-woven fabric is not limited. Single non-woven fabrics of a single fiber or composite fiber made of a material such as rayon, nylons (polyamides), polyesters, polyacrylates, polypropylene, vinylon, polyethylene, polyurethane, cupra, cotton, cellulose, and pulp, or laminates of blended or accumulated fiber layers of such fibers are useful. Furthermore, from the standpoint of production process, dry non-woven fabrics, wet non-woven fabrics, spunbonds, spunlaces, and the like can be used. Non-woven fabrics made of a composite fiber having a core-sheath structure are also useful. A non-woven fabric in the side which is brought into contact with the skin is preferably a napping (fluffy) non-woven fabric. Also, stretchable non-woven fabrics and non-stretchable non-woven fabrics are useful.

The water absorptive raw material is not particularly limited so far as it is a water absorptive film or sheet.

The water absorptive raw material is not particularly limited so far as it has water absorption properties consequently regardless of whether or not the raw material has water absorption properties by itself.

Specific examples thereof include water absorptive foamed films or sheets having water absorption properties (for example, foamed bodies of water absorptive foamed polyurethane, etc.) or papers, non-woven fabrics or woven fabrics formed of a fiber having water absorption properties, non-woven fabrics or woven fabrics containing a fiber having water absorption properties, and water absorptive materials such as water absorptive porous films or sheets. Besides, there are enumerated materials in which regardless of the presence or absence of water absorption properties, a water absorbing agent is contained, impregnated, kneaded, transferred or carried on a foamed film or sheet, a non-woven fabric, a woven fabric or porous film or sheet, thereby imparting or increasing water absorption properties; and materials in which regardless of the presence or absence of water absorption properties, a water absorptive raw material such as water absorptive foamed films or sheets, papers, non-woven fabrics, woven fabrics, and porous films or sheets as cut in a planar shape according to the invention is attached to one side or both sides of the material according to the invention, thereby imparting water absorption properties.

In particular, in the heat generating body of the invention, for the purpose of forming the plane which is brought into contact with the skin into a comfortable plane by imparting water absorption properties against sweat, etc., in order that in the case of sweating, the sweat is absorbed, it is preferable that a packaging material in the plane which is brought into contact with the skin is constituted of a packaging material using a non-woven fabric or a woven fabric containing, as the major component, a water absorptive fiber having a water retention of 20 % or more. Examples of the water absorptive fiber having a water retention of 20 % or more include cottons, silks, hemps, wools, polyacrylonitrile based synthetic fibers, polyamide based synthetic fibers, polyvinyl alcohol based synthetic fibers, acetate fibers, triacetate fibers, and regenerated fibers. In addition, non-woven fabrics having a highly water absorptive polymer held in a non-woven fabric can be used as the non-woven fabric having excellent water absorption properties. Incidentally, non-woven fabrics or woven fabrics containing such a fiber as the major component are relatively good with respect to the feeling against the skin.

In addition, highly water absorptive packaging materials having high absorption properties of sweat can be used as the packaging material. Examples thereof include non-woven fabrics containing a fiber whose surface is coated with a highly water absorptive resin, non-woven fabrics containing a hollow fiber having a number of fine pores on the surface thereof, and non-woven fabrics containing a fiber having a capillary action by forming a number of pouches or plural layers in the cross-sectional shape.

Besides, non-woven fabrics or films having a water absorptive inorganic compound held on a non-sticky surface of a packaging material can be used. Examples thereof include non-woven fabrics resulting from holding a powder (for example, diatomaceous earth, zeolite, and silica gel) on a non-woven fabric and films resulting from holding a relatively large amount of a powder (for example, silica and alumina) on a synthetic resin (for example, polyethylene).

In the invention, as a heat seal material constituting a heat seal layer, a single raw material may be used, or a composite raw material having a heat seal layer may be used. The heat seal material is not limited so far as at least a part thereof can be welded upon heating. Examples thereof include hot melt based resins such as polyolefins (for example, polyethylene and polypropylene) or olefin copolymer resins, ethylene based hot melt resins (for example, ethylene-vinyl acetate copolymer resins and ethylene-acrylic acid ester copolymer resins (for example, ethylene-isobutyl acrylate copolymer resins)), polyamide based hot melt resins, butyral based hot melt resins, polyester based hot melt resins, polyamide based hot melt resins, polyester based hot melt resins, polymethyl methacrylate based hot melt resins, polyvinyl ether based hot melt resins, polyurethane based hot melt resins, polycarbonate based hot melt resins,such as polyvinyl acetate, and vinyl chloride-vinyl acetate copolymers; and films or sheets thereof. Furthermore, in these hot melt based resins or films or sheets thereof, ones having various additives (for example, an antioxidant) compounded therein can be used. In particular, low density polyethylene and polyethylene obtained by using a metallocene catalyst are useful.

In the case of interposing a heat generating composition molded body between a substrate and a covering material, the "temporary adhesion" as referred to in the invention means weak pressure-sensitive bonding or adhesion for the purpose of holding the accommodated heat generating composition molded body until at least the substrate and the covering material are adhered to each other via a sticky layer made of an adhesive and heat sealed.

Furthermore, the "deadhesion" as referred to herein means that in the temporary adhering seal part after heat seal, the heat generating composition in a non-heat sealed region is transferred to the foregoing region, thereby releasing the temporary adhesion.

The temporary adhering seal part is formed via a sticky layer. An adhesive constituting the sticky layer is not limited so far as it is a layer formed of a polymer composition which is tacky at the normal temperature and can be heat sealed after the temporary adhesion.

Furthermore, although the adhesive of the foregoing adhesive layer can be used as the adhesive constituting the sticky layer to be used for the temporary adhesion, a non-hydrophilic adhesive is preferable. As the adhesive constituting the sticky layer, one which is well compatible with the heat seal material constituting the heat seal is preferable, and a melting point of a base polymer of the adhesive is preferably not higher than a melting point of the heat seal material. In particular, hot melt based adhesives are preferable. Furthermore, in the case where the heat seal material is made of an olefin based raw material, preferred examples of the adhesive include olefin based adhesives.

Incidentally, a method for providing a sticky layer for the temporary adhesion is not limited. The sticky layer may be entirely provided or partially or intermittently provided. Examples of its shape include various shapes such as a network-like shape, a stripe-like shape, a dot-like shape, and strip-like shape.

The "air-permeable adjusting material" as referred to in the invention comprises a sectional exothermic part and a sectioned part and covers an exothermic part having a difference of altitude via an adhesive layer, etc., thereby adjusting the air permeability into the sectional exothermic part. That is, in the air permeability adjusting material, by covering the exothermic part by the air-permeable adjusting material while utilizing a difference of altitude between the sectional exothermic part and the sectioned part, a partitioned space is formed in at least a part of the periphery of the sectional exothermic part, thereby adjusting the air permeability between the outside and the sectional exothermic part and also imparting a heat insulating effect.

The air permeability of the air permeability adjusting material is not limited so far as it is able to adjust air retention or air permeability in at least a part of the periphery of the sectional exothermic part. However, it is preferable that the air permeability of the air permeability adjusting material is lower than that on the air-permeable surface of the sectional exothermic part as a covering part for covering the heat generating composition molded body.

Furthermore, a region where the air permeability is higher than that in the covering part for covering the heat generating composition molded body may be provided in a local region of the air-permeable adjusting material, thereby keeping the air permeability of other region lower than that on the air-permeable surface of the sectional exothermic part. In this way, it is possible to control an air passage for air, etc.

The fixing region between the air permeability adjusting material and the exothermic part is not limited so far as the both can be fixed and air can go in and out from at least the periphery of the sectional exothermic part. However, the following can be enumerated.

1) The fixing region is fixed in the both ends of the exothermic part or heat generating body.
2) A space is provided entirely in a substantially central part of the exothermic part, and other exothermic part region is defined as the fixing region.
3) A substantially top part of each sectional exothermic part and a substantially central part of each sectioned part are defined as the fixing region.

Here, as the air permeability adjusting material, any material can be used so far as it is provided with a space which communicates with the outside in the surroundings of the sectional exothermic part. Examples of an air permeability adjusting material having a bonding layer and utilizing a plastic film include PE/adhesive, PP/adhesive, polyester/adhesive, PE/non-woven fabric/air-permeable adhesive, PE/non-woven fabric/PE/adhesive, PE/PET/M/PE/non-woven fabric/air-permeable adhesive, PE/heat seal material, PE/non-woven fabric/heat seal material, PE/non-woven fabric/PE/heat seal material, and PE/polyester/M/PE/non-woven fabric/heat seal material. Here, M represents a metal (for example, aluminum and silver), a semiconductor (for example, silicon oxide, silicon oxynitride, silicon nitride, and aluminum oxide), or a metal oxide, oxynitride or nitride. Furthermore, a portion for placing fixing means such as an adhesive layer and a heat sealing agent layer is not limited, and whether it is provided partially or entirely may be properly determined depending upon the intended purpose.

The bonding layer for fixing the air permeability adjusting material is not limited so far as the air permeability adjusting material can be fixed to the heat generating body and is constituted of a usually used bonding agent or adhesive.

In particular, an adhesive is useful, and the adhesive constituting the foregoing adhesive layer can be used.

Furthermore, a method for providing the bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape. Its thickness is not particularly limited but is in the range of from 5 to 1,000 $\mu$m, preferably from 10 to 500 $\mu$m, and more preferably from 15 to 250 $\mu$m. When the thickness of the bonding layer is less than 5 $\mu$m, a desired adhesive strength may not be possibly obtained. On the other hand, when it exceeds 1, 000 $\mu$m, not only it becomes bulky and becomes worse in feeling for use, but also it becomes worse in economy, and therefore, such is not preferable.

Since the heat generating body of the invention has a bending resistance of not more than 60 mm in at least one direction, it is flexible, has a degree of adhesion to curved bodies to be warmed such as the body and is remarkably convenient. In conventional body warmers, a heat generating composition is accommodated in a flat accommodating bag. Thus, the conventional body warmers were not flexible and problematic in adhesion to curved bodies to be warmed and could not be used in a fitted form. The heat generating body of the invention is an irregular heat generating body. Since the concave of the heat generating body of the invention is flexible, a soft part and a rigid part coexist, and the heat generating body of the invention has flexibility as a whole as in cloths.

Furthermore, by making a bending resistance in one direction different from that in an orthogonal direction thereto, modulation of the bending resistance is revealed depending upon the direction so that handling becomes easy.

Here, as a process for producing a heat cloth in which an absolute value of a difference between bending resistances in the two directions as substantially orthogonal directions becomes maximal, there is enumerated a production process in which a heat generating composition molded body having a size of 120 mm in long side length $\times$ 6 mm in short side length $\times$ 2 mm in height is prepared by force-through molding; 12 pieces of the heat generating composition molded body are laminated substantially in parallel at equal intervals of 10 mm on a substrate made of a laminate of a nylon-made non-woven fabric and a polyethylene film; an air-permeable covering material made of a laminate of a nylon-made non-woven fabric and a polyethylene-made porous film is covered thereon; the surroundings of 2 mm outside the periphery of each of the heat generating composition molded bodies are heat sealed in a width of 4 mm; the outer surroundings of the heat cloth constituted of the respective heat generating composition molded bodies are further heat sealed in a width of 8 mm; and the outer surroundings of the heat cloth are cut while leaving the heat seal, thereby producing a heat cloth. In a heat cloth as produced by this production process, an absolute value of a difference between bending resistances in the two directions as substantially orthogonal directions becomes maximal. One side of the heat cloth is flexible and has adhesion, and the other side is rigid and has nerve. Thus, the heat cloth is very excellent in usefulness.

The outer bag is not limited so far as it is impermeable to air, and it may be made of a laminate. Examples thereof include nylon, polyester and polypropylene films which are subjected to a moisture-proof treatment with OPP, CPP, polyvinylidene chloride, metal oxides (including semiconductors) such as aluminum oxide and silicon oxide, etc., aluminum foils, and aluminum-deposited plastic films. As one example thereof, there is enumerated a heat cloth in which the produced heat cloth is sealed and fixed between two air-impermeable films or sheets.

The fixing means is not limited so far as it has capability for fixing a thermal packaging body for joint surroundings or a material having an exothermic part to a prescribed part.

As the fixing means, an adhesive layer, a hook and eye, a hook and button, a hook and loop fastener such as Velcro, a magnet, a band, a string, and combination thereof can be arbitrarily used.

Incidentally, in the case of a band, fixing means for adjustment may be further constructed by a combination of a hook and loop fastener and an adhesive layer.

Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material. Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for joint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system.

Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-$\alpha$-olefin copolymers, and ethylene-vinyl acetate copolymers), 1,2-polybutadiene based adhesives made of, as a base polymer, 1, 2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an $\alpha$-olefin. Also, polymers resulting from adding a diene (for example, butadiene and isoprene) as other monomer thereto may be employed.

The $\alpha$-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive. Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2

g/m$^2$/day in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 g/m$^2$/day, preferably not more than 1.0 g/m$^2$/day, more preferably not more than 0.5 g/m$^2$/day, and further preferably from 0.01 to 0.5 g/m$^2$/day. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition. Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar. Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and dl-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof.

However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

The heat generating composition is not limited so far as it is a heat generating composition which contains, as essential components, an iron powder, a carbon component, a reaction accelerator and water, does not contain a flocculant aid, a flocculant, an agglomeration aid, a dry binding material, a dry binding agent, a dry binder, an adhesive binder, a thickener and an excipient, contains surplus water so as to have a water mobility value of from 0.01 to 20, has moldability due to the surplus water, with the water in the heat generating composition not functioning as a barrier layer, and is capable of causing an exothermic reaction upon contact with air.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

In addition, if desired, at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof may be further added to the heat generating composition.

Furthermore, in the heat generating composition of the invention or the like, although there is no particular limitation for the compounding ratio thereof, it is preferred to select the compounding ratio such that the amount of the reaction accelerator is from 1.0 to 50 parts by weight, the amount of water is from 1.0 to 60 parts by weight, the amount of the carbon component is from 1.0 to 50 parts by weight, the amount of the water retaining agent is from 0.01 to 10 parts by weight, the water absorptive polymer is from 0.01 to 20 parts by weight, the amount of the pH adjusting agent is from 0.01 to 5 parts by weight, and the amount of the hydrogen formation inhibitor is from 0.01 to 12 parts by weight, respectively based on 100 parts by weight of the iron powder; and that the heat generating composition has a water mobility value of from 0.01 to 20.

In addition, the following components may be added in compounding ratios as described below to the iron powder to the heat generating composition. That is, the amount of the metal other than iron is from 1.0 to 50 parts by weight, the amount of the metal oxide other than iron oxide is from 1.0 to 50 parts by weight, the amount of the surfactant is from 0. 01 to 5 parts by weight, the amount of each of the hydrophobic polymer compound, the aggregate, the fibrous material, the functional substance, the organosilicon compound and the pyroelectric substance is from 0.01 to 10 parts by weight, the amount of each of the moisturizer, the fertilizer component and the heat generating aid is from 0.01 to 10 parts by weight, and the amount of the acidic substance is from 0.01 to 1 part by weight based on 100 parts by weight of the iron powder. Incidentally, a magnetic material may further be compounded, and its compounding ratio may be properly determined depending upon the desire.

Incidentally, these compounding ratios can also be applied in a reaction mixture and a heat generating mixture. Furthermore, a water mobility value of the reaction mixture is usually less than 0.01.

As the water, one from a proper source may be employed. Its purity and kind and the like are not particularly limited.

In the case of the heat generating composition, the content of water is preferably from 1 to 70 % by weight, more preferably from 1 to 60 % by weight, further preferably from 7 to 60 % by weight, still further preferably from 10 to 50 % by weight, and even further preferably from 20 to 50 % by weight of the heat generating composition.

Furthermore, in the case of the reaction mixture or heat generating mixture prior to the contact treatment with an oxidizing gas, the content of water is preferably from 0.5 to 20 % by weight, more preferably from 1 to 20 % by weight, further preferably from 3 to 20 % by weight, and still further preferably from 4 to 15 % by weight of the reaction mixture or heat generating mixture.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.

Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, *shirasu* balloon, and *taisetsu* balloon). In order to increase a water retaining ability and enhance a shape holding ability of such a water retaining agent, the water retaining agent may be subjected to a processing treatment such as baking and/or pulverization.

The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.

Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth)acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly (meth) acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials. The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, $NaOH$, $KOH$, $Ca(OH)_2$, $Mg(OH)_2$, and $Ca_3(PO_4)_2$.

The hydrogen formation inhibitor is not limited so far as it is able to inhibit the formation of hydrogen. Examples thereof include one member or two or more members selected from the group consisting of sulfur compounds, oxidizing agents, alkaline substances, sulfur, antimony, selenium, phosphorus, and tellurium. Incidentally, examples of sulfur compounds include compounds with an alkali metal or an alkaline earth metal, metal sulfides such as calcium sulfide, metal sulfites such as sodium sulfite, and metal thiosulfates such as sodium thiosulfate.

Examples of the oxidizing agent include nitrates, oxides, peroxides, halogenated oxygen acid salts, permanganates, and chromates.

The aggregate is not limited so far as it is useful as a filler and/or is useful for making the heat generating composition porous. Examples thereof include fossilized coral (for example, coral fossil and weathered coral fossil), bamboo charcoal, *bincho* charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder. Incidentally, it is to be noted that kaolin and crystalline cellulose are not contained in the heat generating composition of the invention.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances. The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon. The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenol-ethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

As the metal oxide other than iron oxide, any material can be employed so far as it does not hinder the oxidation of iron by an oxidizing gas, and examples thereof include manganese dioxide and cupric oxide.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

As the "iron powder" as referred to herein, usual iron powders, iron alloy powders and active iron powders such as iron powders comprising particles, a surface of each of which is at least partially covered with an oxygen-containing film , and iron alloy powders comprising particles , a surface of each of which is at least partially covered with an oxygen-containing film, are preferable. Incidentally, the "iron oxide film" as referred to herein is a film made of oxygen-containing iron such as iron oxide, hydroxide or oxyhydroxide. Furthermore, the "active iron powder" as referred to herein is a powder in which an iron oxide film is formed at least locally on the surface of an iron powder, from which an oxidation reaction promoting effect is obtained by a local cell as formed between an iron matrix and an iron oxide film or a pit inside and outside the iron oxide film.

The iron powder is not limited, and examples thereof include cast iron powders, atomized iron powders, electrolyzed iron powders, reduced iron powders, sponge iron powders, and iron alloy powders thereof. In addition, the iron powder may contain carbon or oxygen, and an iron powder containing 50 % or more of iron and other metals may be employed. The kind of the metal which is contained as an alloy, etc. is not particularly limited so far as the iron component works as a component of the heat generating composition. Examples of such a metal include metals such as aluminum, manganese, copper, nickel, silicon, cobalt, palladium, and molybdenum, and semiconductors. The metal of the invention includes a semiconductor. Such a metal or alloy may be contained only in the surface or the interior, or may be contained in both the surface and the interior.

In the iron powder of the invention, the content of the metal other than iron is usually from 0.01 to 50 % by weight, and preferably from 0.1 to 10 % by weight based on the whole of the iron powder.

Examples of the iron powder having an oxygen-containing film on at least a part of the surface of the iron include:

(A) an active iron powder in which the surface of an iron component is at least partially oxidized, which is obtained by contact treating the essential components of the heat generating composition or the essential components to which acidic substances or other necessary components are added with an oxidizing gas, thereby partially oxidizing the iron component;

(B) an active iron powder in which the content of wustite is from 2 to 50 % by weight in terms of an X-ray peak intensity ratio to iron;

(C) an iron powder having an iron oxide film having a thickness of 3 nm or more on the surface thereof; and

(D) a mixture of an active iron powder and an iron powder other than an active iron powder.

With respect to (A), although the mechanism is not elucidated in detail, it is assumed that upon contact between the oxidizing gas and the components, not only an iron oxide film, namely, an oxygen-containing film is formed on the surface of the iron powder due to the oxidation of the components, especially the oxidation of the iron powder, but also the surface of active carbon is oxidized and/or the oxidized iron component is adhered, whereby hydrophilicity is imparted or improved, and coupling between the components or structurization takes place through the mediation of water.

That is, it is assumed that some kind of a change in the function occurs such that an iron oxide film is formed on the surface of the iron powder, the shape of the iron powder particle becomes irregular, a strain is generated due to the oxidation, or a water-containing pit is formed, whereby the iron powder is activated and exothermic rising properties are improved.

Furthermore, the case where magnetite ($Fe_3O_4$) is present in the iron oxide film is preferable because the conductivity is excellent, and the case where hematite ($Fe_2O_3$) is present in the iron oxide film is also preferable because the iron oxide film becomes porous. Moreover, it is assumed that the carbon component is oxidized on the surface thereof and becomes a carbon component which is rich in oxides on the surface thereof, whereby the hydrophilicity increases and the activity increases.

The thickness of the iron oxide film which is an oxygen-containing film covering the surface of the iron powder, as measured by the Auger electron spectroscopy, is usually 3 nm or more, preferably from 3 nm to 100 $\mu$m, more preferably from 30 nm to 100 $\mu$m, further preferably from 30 nm to 50 $\mu$m, still further preferably from 30 nm to 1 $\mu$m, even further preferably from 30 nm to 500 nm, and even still further preferably from 50 nm to 300 nm.

When the thickness of the oxygen-containing film of iron is 3 nm or more, the thickness of the oxygen-containing film of iron is able to exhibit a promoting effect of the oxidation reaction, and upon contact with an oxidizing gas such as air, is able to immediately initiate the oxidation reaction. When the thickness of the oxygen-containing film of iron is 100 $\mu$m or more, though the heat generation time may possibly be shortened, such is applicable depending upon the utility.

Furthermore, according to the active iron powder, by using a reaction mixture containing, as essential components, an iron powder, a reaction accelerator and water and having a water content of from 0. 5 to 20 % by weight and a water mobility value showing a surplus water content of less than 0.01, the reaction rate at the time of the contact treatment with an oxidizing gas can be raised, thereby achieving a time required for regulating a temperature rise of the reaction mixture at 1 °C or more within 10 minutes. By shortening a time required for arrival at a prescribed temperature or higher, proper activation can be achieved, and unnecessary oxidation on the iron powder can be prevented.

Furthermore, the heat generating composition prepared by adding a carbon component, etc. to a heat generating mixture as produced by contact treating the reaction mixture with an oxidizing gas or adjusting the water content so as to have a water mobility value of from 0.01 to 50 is properly tacky, has excellent moldability and is able to be applied with a molding method such as a force-through die molding method and a cast molding method, whereby heat generating bodies of various shapes can be produced. In particular, a heat generating composition having a water mobility value of from 0.01 to 20 is excellent because it initiates an exothermic reaction immediately after contacting with air, has excellent exothermic rising properties and has excellent moldability.

The contact treatment method of the reaction mixture with an oxidizing gas is not particularly limited so far as it is able to contact treat a reaction mixture containing, as essential components, an iron powder, a reaction accelerator and water and having a water content of from 0.5 to 20 % by weight and a water mobility value of less than 0.01 with an oxidizing gas and regulate a temperature rise of the reaction mixture at 1 °C or more.

Specific examples thereof include:

(1) a process for producing a heat generating mixture containing an iron powder having an iron oxide film on the surface thereof by subjecting a reaction mixture of an iron powder, a reaction accelerator and water in an oxidizing gas atmosphere to a self-exothermic reaction, thereby partially oxidizing the iron powder;
(2) a process for producing a heat generating mixture by subjecting a reaction mixture of an iron powder, a reaction accelerator, an acidic substance and water in an oxidizing gas atmosphere to a self-exothermic reaction;
(3) a process for producing a heat generating mixture by subjecting a reaction mixture of an iron powder, a reaction accelerator, a carbon component and water in an oxidizing gas atmosphere to a self-exothermic reaction;
(4) a process for producing a heat generating mixture by subjecting a reaction mixture of an iron powder, a reaction accelerator, an acidic substance, a carbon component and water in an oxidizing gas atmosphere to a self-exothermic reaction;
(5) a process for producing a heat generating mixture containing a partially oxidized iron powder by carrying out the method as set forth above in any one of (1) to (4), wherein the reaction mixture or heat generating mixture as set forth above in any one of (1) to (4) contains a component other than the foregoing components;
(6) a process for producing a heat generating mixture by carrying out the method as set forth above in any one of (1) to (5) under circumstances heated so as to have temperature of at least 10 °C higher than the circumferential temperature;
(7) a process for producing a heat generating mixture by carrying out the method as set forth above in any one of (1) to (6) by blowing an oxidizing gas;
(8) a process for producing a heat generating mixture by carrying out the method as set forth above in (7) by blowing the oxidizing gas heated so as to have a temperature of at least 10 °C higher than the circumferential temperature;

(9) a process for producing a heat generating composition by carrying out the method as set forth above in any one of (1) to (8) by contact treating with an oxidizing gas until the temperature exceeds a maximum temperature which is a maximum point of temperature rise by the exothermic reaction;

(10) a process for producing a heat generating mixture by carrying out the method as set forth above in any one of (1) to (8) by contact treating with an oxidizing gas until the temperature exceeds a maximum temperature by the exothermic reaction and drops by at least 10 to 20 °C from the maximum temperature;

(11) a process for producing a heat generating composition by carrying out the method as set forth above in any one of (1) to (8) by contact treating with an oxidizing gas until the temperature exceeds a maximum temperature which is a maximum point of temperature rise by the exothermic reaction and after intercepting the oxidizing gas, holding it until the temperature of at least the reaction mixture drops by at least 10 to 20 °C from the maximum temperature; and

(12) a process for producing a heat generating mixture by heating the reaction mixture or heat generating mixture as set forth above in any one of (1) to (5) under oxidizing gas circumstances while regulating a temperature rise at 1 °C or more.

In addition, a heat generating mixture as prepared by adding other components to the heat generating mixture and further treating with an oxidizing gas may be employed.

Incidentally, the circumstances of the reaction mixture at the time of contact treatment with an oxidizing gas are not limited so far as the reaction mixture is brought into contact with an oxidizing gas under circumstances of 0 °C or higher and a temperature rise of the reaction mixture is regulated at 1 °C or more within 10 minutes. In the case where the contact treatment is carried out in an open system, the circumstances may be either the state that the reaction mixture is present in a lid-free vessel or the state that an oxidizing gas such as air comes into a vessel through an air-permeable sheet-like material such as non-woven fabrics.

Furthermore, the contact treatment with an oxidizing gas may be carried out with or without stirring in a fluidized or non-fluidized state and may be carried out in a batch or continuous system.

Examples of the final heat generating composition include:

1) a heat generating composition containing, as a heat generating composition raw material, a heat generating mixture produced in the process as set forth above in any one of (1) to (12);

2) a heat generating composition obtained by adding other components to the heat generating composition as set forth above in 1); and

3) a heat generating composition obtained by adjusting the water content of the heat generating composition as set forth above in 1) or 2).

The order of the timing of adding other components than the essential components and the timing of adjusting the water content is not limited.

Here, the water content in the reaction mixture and also the heat generating mixture prior to the treatment with an oxidizing gas is usually from 0.5 to 20 % by weight, preferably from 1 to 15 % by weight, more preferably from 2 to 10 % by weight, further preferably from 3 to 10 % by weight, and still further preferably from 6 to 10 % by weight.

The temperature of the reaction mixture after the contact with an oxidizing gas is not limited so far as the temperature rise is regulated at 1 °C or more. The temperature of the reaction mixture after the contact with an oxidizing gas is preferably from 1 to 80 °C, more preferably from 1 to 70 °C, further preferably from 1 to 60 °C, and still further preferably from 1 to 40 °C.

The circumferential temperature at the time of contact between the reaction mixture and the oxidizing gas is not limited so far as the temperature of the reaction mixture is raised to a prescribed temperature or higher. The circumferential temperature at the time of contact between the reaction mixture and the oxidizing gas is preferably 0 °C or higher, more preferably from 0 to 250 °C, further preferably from 10 to 200 °C, still further preferably from 20 to 150 °C, even further preferably from 25 to 100 °C, and even still further preferably from 25 to 50 °C.

The time of contact between the reaction mixture and the oxidizing gas is not limited so far as the time required for regulating a temperature rise at 1 °C or more is within 10 minutes. The time of contact between the reaction mixture and the oxidizing gas is preferably from one second to 10 minutes, more preferably from one second to 7 minutes, further preferably from one second to 5 minutes, still further preferably from 2 seconds to 5 minutes, even further preferably from 2 seconds to 3 minutes, and even still further preferably from 2 seconds to one minute.

The temperature of the oxidizing gas is not limited so far as the foregoing circumferential temperature is kept.

As the "oxidizing gas" as referred to herein, any gas can be used as the oxidizing gas so far as it is oxidizing. Examples thereof include an oxygen gas, air, and mixed gases of an inert gas (for example, a nitrogen gas, an argon gas, and a helium gas) and an oxygen gas. Although the mixed gas is not limited so far as it contains oxygen, mixed gases containing 10 % or more of an oxygen gas are preferable, and of these, air is especially preferable. If desired, a catalyst such as platinum, palladium, iridium, and compounds thereof can also be used.

The oxidation reaction can be carried out under stirring in an oxidizing gas atmosphere optionally under a pressure and/or upon irradiation of ultrasonic waves.

The optimal condition of the oxidation reaction may be properly experimentally determined.

An amount of the oxidizing gas to be used is not limited but may be adjusted depending upon the kind of the oxidizing gas, the kind and particle size of the iron powder, the water content, the treatment temperature, the treatment method, and the like.

In the case of an open system, there is no limitation so far as a necessary amount of oxygen can be taken in. In order to prevent fly of the reaction mixture or contamination of dusts, etc., the system may be surrounded by an air-permeable raw material such as non-woven fabrics and woven fabrics. So far as the system is in an air-permeable state, it is to be noted that the system is an open system.

In the case where air is used in the system of blowing an oxidizing gas, for example, the amount of air is preferably from 0.01 to 1,000 L/min, more preferably from 0.01 to 100 L/min, and further preferably from 0.1 to 50 L/min per 200 g of the iron powder under one atmosphere. In the case of other oxidizing gas, the amount of the oxidizing gas may be converted on the basis of the case of air.

If desired, a peroxide may be added. Examples of the peroxide include hydrogen peroxide and ozone.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of the contact treatment with a mixed oxidizing gas at the time of adjusting the water content are not limited, and examples thereof include those in an oxidizing gas atmosphere and those in blowing of an oxidizing gas.

A method for measuring a temperature rise of the heat generating composition is as follows.

1) A heat generating composition is allowed to stand in a state that it is sealed in an air-impermeable outer bag for one hour under a condition that the circumferential temperature is 20 ± 1 °C.
2) A magnet is provided in the vicinity of a central part of the back side of a polyvinyl chloride-made supporting plate (3 mm in thickness × 600 mm in length × 600 mm in width) of a footed supporting table so as to cover a cavity shape of a molding die.
3) A temperature sensor is placed on the central part of the supporting plate.
4) A polyethylene film (25 μm in thickness × 250 mm in length × 200 mm in width) as provided with an adhesive layer having a thickness of about 80 μm is stuck onto the supporting plate via a sticky layer such that the center of the polyethylene film is positioned at the sensor.
5) The heat generating composition is taken out from the outer bag.
6) A template (250 mm in length × 200 mm in width) having a cavity (80 mm in length × 50 mm in width × 3 mm in height) is placed above the central part of the polyethylene film; a sample is placed in the vicinity of the cavity; a force-in die plate is moved along the template; the sample is charged into the cavity while stuffing; and the sample is leveled while stuffing along the template plane (force-in die molding), thereby filling the sample in the die. Next, the magnet beneath the supporting plate is removed, and the temperature measurement is started.

With respect to the measurement of the exothermic temperature, the temperature is measured for 10 minutes at a measurement timing of 2 seconds using a data collector, and exothermic rising properties are judged in terms of the temperature after elapsing 3 minutes.

The heat generation test of the heat generating body follows the JIS temperature characteristic test.

In the iron powder or active iron powder in the oxidizing gas-treated heat generating composition, at least a part of the surface thereof is covered by an oxygen-containing film of iron. The degree of covering on the surface of the oxygen-containing film of iron is not limited so far as at least a part of the surface thereof is covered, and the surface may be entirely covered. In the case of the heat generating composition of the invention, since an ion of the reaction accelerator such as a chlorine ion is contained in the heat generating composition, there is no corrosion effect of the oxide film due to anti-corrosion effect by the ion of the reaction accelerator such as a chlorine ion. Thus, the oxidation reaction which is a sort of corrosion is not hindered. In particular, in the case where an oxygen-containing film of iron is prepared while the ion of the reaction accelerator such as a chlorine ion exists together, the subject effect is large. In the case where a metal other than iron is present on the surface, it is only required that at least other part of the metal portion other than iron is covered by the oxygen-containing film of iron.

In the iron powder of the invention, not only a region where (1) entire (uniform) corrosion, (2) pitting or crevice corrosion, (3) stress corrosion cracking, or the like is generated, but also irregularities or crevices are formed. For that reason, it is assumed that the iron powder of the invention has hydrophilicity and oxidation catalytic properties (FeO, etc.) in its own portion. In producing the heat generating composition, it is important that the iron powder has an oxygen-containing film in its own portion without relying upon mixing. In particular, in the iron component as prepared by contact treating the iron component and the reaction accelerator and water as essential components with an oxidizing gas, it is thought that a reaction active part composed mainly of an oxide, a hydroxide, a chlorine ion, a hydrogen ion, etc. is formed, whereby exothermic reactivity and hydrophilicity are improved and exothermic rising properties and moldability are remarkably improved.

With respect to (B), the amount of FeO (wustite) which is contained in the iron component containing a prescribed amount of wustite is usually from 2 to 50 % by weight, preferably from 2 to 40 % by weight, more preferably from 2 to 30 % by weight, further preferably from 5 to 30 % by weight, and still further preferably from 6 to 30 % by weight in terms of an X-ray peak intensity ratio of iron. When the amount of FeO (wustite) exceeds 50 % by weight, though the exothermic rising properties are good, the duration of heat generation becomes short. On the other hand, when it is less than 2 % by weight, the exothermic rising properties become dull.

The thickness of the oxygen-containing film of a prescribed amount or the oxygen-containing film of iron powder containing wustite and the amount of wustite are applied to the heat generating composition or the heat generating composition molded body at the time of lamination.

An iron powder containing a carbon component and/or covered by a carbon component is also preferable. Although a proportion of the carbon component is not limited so far as a ratio of the iron component to the carbon component is 50 % by weight or more, an iron powder in which the surface thereof is partially covered by from 0.3 to 3.0 % by weight of a conductive carbonaceous substance is useful. Examples of the conductive carbonaceous substance include carbon black, active carbon, carbon nanotubes, carbon nanohorns, and flullerenes. Ones which have become conductive by doping are also employable. Examples of the iron powder include reduced iron powders, atomized iron powders, and sponge iron powders. In particular, the case where the conductive carbonaceous substance is active carbon and the iron powder is a reduced iron powder is useful as a heat generating body.

Furthermore, in order to efficiently carry out covering by a conductive carbonaceous substance, an oil such as a spindle oil may be added in an amount of from 0.01 to 0.05 % by weight to such an extent that the fluidity of the iron powder is not hindered.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:

The heat generating composition is taken out from the heat generating body and measured according to the foregoing method of measuring a water mobility value.

2) Thickness and amount of wustite of iron oxide film of iron powder:

A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

The heat generating composition of the invention contains, as essential components, an iron powder, a carbon component, a reaction accelerator and water, and its production process is one which can be put into practical use on an industrial scale. A reaction mixture containing, as essential components, an iron powder, a reaction accelerator and water and having a water content of from 1 to 20 % by weight and a water mobility value showing a surplus water content of less than 0.01 is brought into contact with an oxidizing gas under circumstances at 0 °C or higher, a temperature rise of the reaction mixture is regulated at 1 °C or more within 10 minutes to produce a heat generating mixture, and the subject heat generating mixture is used as a raw material to form a heat generating composition. Alternatively, a heat generating composition may be formed by subsequently further adjusting the water content, or by further adding a carbon component, etc. and adjusting the water content.

In the invention, it has become possible to realize the contact treatment with an oxidizing gas within a short period of time by regulating the water content of the reaction mixture at a fixed amount or less, especially regulating the surplus water content of the reaction mixture at a fixed amount or less and carrying out an oxidizing contact treatment. By specifying the surplus water content and performing the treatment within a short period of time, adverse influences such as poor initial exothermic rising of the heat generating composition and shortening of the heat generation-retaining time can be avoided. Thus, it has become possible to establish an industrial mass-production process. Furthermore, although stirring or the like may not be achieved during the contact treatment with an oxidizing gas, when stirring or the like is achieved, the contact treatment with an oxidizing gas can be surely carried out.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of mixing at the time of adjusting the water content are not limited, and examples thereof include those in an oxidizing gas atmosphere and those in blowing of an oxidizing gas.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

The heat generating composition of the invention contains, as essential components, an iron powder, a carbon component, a reaction accelerator and water and is started from a mixture obtained by contact treating a reaction mixture containing, as essential components, an iron powder, a reaction accelerator and water with an oxidizing gas. The heat generating composition of the invention is usually one obtained by adjusting the water content of a heat generating mixture and is a heat generating composition which is satisfactory in the exothermic rising, has a suitable amount of surplus water and has excellent moldability. Furthermore, it is possible to produce a heat generating body which can become promptly warm at the time of use.

Accordingly, at least the iron powder further including the carbon component has a history of oxidation by the contact treatment with an oxidizing gas, and it is thought that this is deeply related to excellent exothermic rising properties, exothermic endurance and excellent moldability.

When the iron powder which is contact treated with an oxidizing gas according to the invention is used, the amount of addition of the carbon component (for example, active carbon) in the heat generating composition can be reduced by, for example, 20 % or more. By reducing the amount of addition of the carbon component, the costs are lowered.

According to the production process of the heat generating mixture of the invention, it is possible to obtain a heat generating composition having excellent exothermic rising properties, excellent hydrophilicity, and excellent moldability. In particular, a heat generating composition having remarkably excellent moldability and exothermic characteristics together can be obtained while specifying the water availability value at from 0.01 to 50, in particular 0.01 to 20.

The heat generating composition as produced by the production process of the invention is remarkably improved with respect to exothermic rising properties. Thus, the amount of addition of the carbon component (such as active carbon) in the heat generating composition can be reduced by, for example, 20 % or more so that it can contribute to a reduction in costs.

Furthermore, since the hydrophilicity is remarkably improved, the moldability with a mold is remarkably improved. Thus, since after molding, collapsed pieces of the heat generating composition are not scattered on the surroundings of the heat generating composition molded body, sealing can be appropriately achieved so that a heat generating body free from sealing cut can be produced. In this way, heat generating composition molded bodies of various shapes can be produced, and heat generating bodies of various shapes are formed.

Furthermore, in view of improving the exothermic rising properties of the heat generating composition, the following are preferable.

1) A heat generating composition obtained by a contact treatment (self heat generation) of a mixture of the essential components of the heat generating composition, or a mixture of the foregoing mixture and an acidic substance or other necessary components with an oxidizing gas, a heat generating composition obtained by additionally adjusting the water content of the foregoing heat generating composition, or a heat generating composition obtained by adding and mixing other components in the foregoing heat generating composition.

2) Any one of the following active iron powders having an oxygen-containing film (for example, oxides) on at least a part of the surface thereof is used as the iron powder: (a) an iron powder having an oxygen-containing film of iron having a thickness, as measured by the Auger electron spectroscopy, of 3 nm or more on the surface thereof and (b) an iron powder having a content of wustite of from 2 to 50 % by weight in terms of an X-ray peak intensity ratio to iron.

3) A mixture of an active iron powder having an oxygen-containing film (for example, oxides) on at least a part of the surface thereof and an iron powder not having an oxygen-containing film is used as the iron powder. In this case, a mixture containing 60 % by weight or more of an active iron powder and less than 40 % by weight of an iron powder other than the active iron is preferable.

In the case of storing the heat generating composition which is treated with an oxidizing gas or the heat generating composition containing an active iron powder, or a material utilizing the same over a long period of time, it is preferred to combine a hydrogen formation inhibitor therewith. This is because in this way, a heat generating body having excellent exothermic characteristics, which is inhibited in the formation of hydrogen, is free from swelling of the outer bag at the time of storage, etc. and has satisfactory exothermic rising properties, is obtained.

Furthermore, so far as the rising characteristics are not affected, the heat generating composition having a water mobility value falling outside the range of from 0.01 to 20 can contain a water-soluble polymer, a flocculant aid, a flocculant, an agglomeration aid, a dry binding material, a dry binding agent, a dry binder, an adhesive raw material, a tackifier, an excipient, a flocculating agent, or a soluble sticky raw material.

Furthermore, since a marketed heat generating body in which a heat generating composition is accommodated in an accommodating bag is provided on the assumption that it is accommodated in an outer bag which is an air-impermeable accommodating bag and is storable over a long period of time, it is preferred to use a heat generating composition containing a hydrogen formation inhibitor. Since the heat generating composition which has passed through the contact treatment with an oxidizing gas is an active composition, it is important that the heat generating composition contains a hydrogen formation inhibitor. Also, this efficacy is further strengthened by using a pH adjusting agent together.

Furthermore, so far as the reaction characteristics and exothermic characteristics are not affected, the heat generating composition having a water mobility value of less than 0.01 may contain a flocculant aid, a flocculant, an agglomeration aid, a dry binder, a dry binding agent, a dry binding material, a sticky raw material, a thickener, an excipient, or a water-soluble polymer in an amount ranging from 0.01 to 3 parts by weight respectively.

The "flocculant aid" as referred to herein is a flocculant aid as described in Japanese Patent No. 3,161,605 (JP-T-11-508314) such as gelatin, natural gum, and corn syrup.

The "flocculant" as referred to herein is a flocculant as described in JP-T-2002-514104 such as corn syrup and maltitol syrup.

The "agglomeration aid" as referred to herein is an agglomeration aid as described in JP-T-2001-507593 such as corn syrup.

The "dry binder" as referred to herein is a dry binder as described in JP-T-2002-514104 such as microcrystalline cellulose, maltodextrin, and mixtures thereof.

The "dry binding agent" as referred to herein is a dry binding agent as described in JP-T-2001-507593 such as maltodextrin and sprayed lactose.

The "dry binding material" as referred to herein is a dry binding material as described in JP-T-11-508314 such as microcrystalline cellulose, maltodextrin, and mixtures thereof.

The "sticky raw material" or the "binder" as referred to herein is a sticky raw material or binder as described in JP-A-4-293989 such as water glass, polyvinyl alcohol (PVA), and carboxymethyl cellulose (CMC).

The "thickener" as referred to herein is a thickener as described in JP-A-6-343658 such as corn starch and potato starch.

The "excipient" as referred to herein is an excipient as described in JP-A-7-194641 such as $\alpha$-starch and sodium alginate.

As the "water-soluble polymer" as referred to herein, the water-soluble polymer in the adhesive layer can be used.

The particle size of the water-insoluble solid component constituting the moldable heat generating composition of the invention is not limited so far as the heat generating composition has moldability. In the case where any one of length, width and height as the size of the heat generating composition molded body as molded from the heat generating composition is small, the moldability is improved by making the particle size small.

In addition, it is preferable in view of molding that the particle size of the solid component constituting the moldable heat generating composition is small. A maximum particle size of the water-insoluble solid component exclusive of the reaction accelerator and water in the components constituting the moldable heat generating composition is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.

Incidentally, with respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component. Incidentally, each of the mesh sieves may be combined with other mesh sieves. Here, the particles which have passed

**EP 1 782 777 A1**

through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300 $\mu$m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.

Furthermore, the heat generating composition can be classified into a powder, a granulate heat generating composition (having a water mobility value of less than 0.01), a moldable heat generating composition (having a water mobility value of from 0.01 to 20), and a sherbet-like heat generating composition (having a water mobility value exceeding 20 but not more than 50) depending upon the state of adjustment of the water content or surplus water. The heat generating composition as classified depending upon the water mobility value is as described previously.

The "moldability" as referred to in the invention exhibits that a laminate of the heat generating composition having a cavity or concave die shape can be formed by force-through molding using a trimming die having a cavity or cast molding using a concave die and after molding including mold release, the molding shape of the heat generating composition molded body is held. When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, sealing can be achieved without causing cutting in seal. The presence of the spots causes insufficient sealing.

Next, with respect to the moldability, a measurement device, a measurement method and a judgment method will be described below.

1) Measurement device:

With respect to the measurement device, a stainless steel-made molding die (a plate having a size of 2 mm in thickness $\times$ 200 mm in length $\times$ 200 mm in width and having a cavity as treated by R5 in four corners of 60 mm in length $\times$ 40 mm in width in a central part thereof) and a fixable leveling plate are disposed above a travelable endless belt, and magnets (two magnets having a size of 12.5 mm in thickness $\times$ 24 mm in length $\times$ 24 mm in width are disposed in parallel) are disposed under the endless belt. The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness x 200 mm in length $\times$ 200 mm in width is placed on the endless belt of the measurement device, a polyethylene film having a size of 70 $\mu$m in thickness $\times$ 200 mm in length $\times$ 200 mm in width is placed thereon, and a stainless steel-made molding die is further placed thereon. Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8 m/min.

After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 $\mu$m is not present and the number of collapsed pieces of the heat generating composition molded body having a maximum length of from 300 to 800 $\mu$m is not more than 5, it is to be noted that the heat generating composition has moldability. The moldability is an essential property for a heat generating composition to be used in the molding system. If the heat generating composition does not have moldability, it is impossible to produce a heat generating body by the molding system.

The heat generating composition of the invention has resistance to compression. The "resistance to compression" as referred to herein means that a heat generating composition compressed body obtained by compressing a heat generating composition molded body as accommodated in a molding die within the die to such an extent that the thickness is 70 % of the die thickness holds 80 % or more of exothermic rising properties of the exothermic rising properties of the heat generating composition molded body before compression (a difference in temperature between one minute and 3 minutes after starting a heat generation test of the heat generating composition).

Here, the measurement method of exothermic rinsing properties for the resistance to compression will be described below.

1. Heat generating composition molded body:

1) A magnet is provided in the vicinity of a central part of the back side of a polyvinyl chloride-made supporting plate (3 mm in thickness × 600 mm in length × 600 mm in width) of a footed supporting table so as to cover a cavity shape of a molding die.
2) A temperature sensor is placed on the central part the surface of the supporting plate.
3) A polyethylene film (25 μm in thickness × 250 mm in length × 200 mm in width) as provided with an adhesive layer having a thickness of about 80 μm is stuck onto the supporting plate via a sticky layer such that the center of the polyethylene film is positioned at the sensor.
4) On an underlay plate (280 mm in length × 150 mm in width × 50 μm to 2 mm in thickness), a polyethylene film (230 mm in length × 155 mm in width × 25 μm to 100 μm in thickness) is placed such that one end of the polyethylene film is projected by about 20 mm outside the underlay plate and that one end thereof in the length direction is substantially coincident with one end of the underlay plate.
5) A template (230 mm in length × 120 mm in width × 3 mm in thickness) having a cavity (80 mm in length × 50 mm in width × 3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.
6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.
7) Next, the magnet beneath the supporting plate is removed; the end portion of the projected polyethylene film is pressed; the underlay plate is removed; and the temperature measurement is started.

2. Heat generating composition compressed body:

1) to 6) are the same as in the case of the heat generating composition molded body.
8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.
9) The resulting assembly is placed on the supporting plate together with the underlay plate; the magnet beneath the supporting plate is removed; the end portion of the projected polyethylene film is pressed; the underlay plate is removed; and the temperature measurement is started.

With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.
The thickness after compression is preferably from 50 to 99.5 %, more preferably from 60 to 99.5 %, and further preferably from 60 to 95 % of the die thickness.
Incidentally, in the invention, it is to be noted that the heat generating composition molded body includes a heat generating composition compressed body.

The process for producing a heat cloth of the invention is a process for producing a heat cloth by laminating a heat generating composition molded body on a substrate by a molding system, further covering a covering material thereon, and sealing the surroundings of the heat generating composition molded body to provide a sectional exothermic part constituted of the heat generating composition molded body between packaging materials which constitute an air-permeable accommodating bag, whereby two or more plural sectional exothermic parts are disposed and fixed at intervals and an exothermic part is formed of a gathering of the sectional exothermic parts, which is characterized in that a maximum width of the sectional exothermic parts is from 1 to 20 mm; a maximum diameter (in the case where two or more axes are present as in an ellipse, the maximum diameter means a shortest axis such as a minor axis) is from 1 to 20 mm; a maximum height is from 0.1 to 20 mm; a space between the sectional exothermic parts is from 1 to 20 mm; the heat generating composition contains, as essential components, an exothermic substance, a carbon component, a reaction accelerator and water and has a water mobility value of from 0.01 to 20; 80 % or more of water-insoluble solid components which constitute the heat generating composition have a particle size of not more than 300 m and a maximum particle size of not more than 1 mm; the packaging material is made of the substrate and the covering material; at least one or a part of the substrate and the covering material is permeable to air; and at least the surroundings of the heat cloth are sealed. In addition, each of the substrate and the covering material has a heat seal layer; an adhesive layer made of an adhesive is provided on at least one of the heat seal layers; in the substrate, the heat generating molded body and the covering material, the substrate and the covering material are temporarily adhered via the sticky layer in at least the periphery of the heat generating composition molded body; and after forming a temporary adhering seal part, the temporary adhering seal part is heat sealed to form a heat seal part. Furthermore, heat sealing is carried out in a width narrower than that of the temporary adhering seal part, and thereafter, the heat generating composition is moved into a region which is not heat sealed within the temporary adhering seal part, thereby achieving deadhesion. In this way, the heat generating composition molded body becomes stable; real sealing by heat sealing becomes easy; seal deviation or the like does not occur; a heat seal width with fine lines can be embodied at high speed without causing sealing; and it is possible to section an exothermic part without causing a lowering of exothermic characteristics such as a decrease in exothermic time due to sectioning of the exothermic part.

As a preferred production process of a heat cloth having sectional exothermic parts in which an exothermic part thereof is sectioned according to the molding system of the invention, any molding method using a die may be employed. Examples thereof include a force-through molding method and a cast molding method.

If desired, the heat generating composition or the heat generating composition molded body may be subjected to in-die compression or out-die compression. The "in-die compression" as referred to herein means that the heat generating composition is compressed by flexible rubber rolls or the like while the heat generating composition is present within the die; and the "out-die compression" as referred to herein means that after the heat generating composition leaves from the die to become a heat generating composition molded body, the heat generating composition molded body is compressed by rolls or the like. Though this compression is usually carried out after covering the heat generating composition molded body by an underlay material and/or a covering material, this may not be carried out.

The heat cloth of the invention is produced through the accommodating step and the subsequent sealing step and cutting step and so on. With respect to the sealing step and the cutting step and so on, conventional methods and devices may be properly selected and used.

Furthermore, in the seal step, the seal is not limited so far as seal is possible. Usually, heat seal or compression seal or a mixture thereof is employed. The surface of the seal part may be of a plain shape or a patterned shape whose cross-sectional shape is irregular, and a mixture of a plain shape and a patterned shape whose cross-sectional shape is irregular. The mixture of pattern as referred to herein means a mixture of a plain shape in the inside of the seal part and a patterned shape in the outside of the seal part, or a mixture of a patterned shape in the inside of the seal part and a plain shape, a partially plain shape or a partially patterned shape in the outside of the seal part. Furthermore, the back side may be plain, with the front side being patterned, and vice versa. Furthermore, a part or the whole of the pattern may be a double pattern. Accordingly, following this, a plain or patterned seal roll is used as a seal roll. Furthermore, a pair of seal rolls may be used. Multiplex seal may be carried out by placing plural seal rolls of two or more. Examples of the multiplex seal include duplex seal, triplet seal, quadruplet seal, and quintuplet seal. The width of seal may be the same or different and may be properly determined. In the case of high-speed seal, a higher number of multiplex seal is preferable. In the case of using a seal roll or a compression seal roll to which the temperature is applied, the temperature of a pair of rolls may be the same, or the temperature of one roll may be different from that of the other roll.

The "force-through die molding" as referred to herein means a continuous formation method in which by using a molding machine for using a molding die and laminating a heat generating composition molded body having a shape of the molding die on a longitudinal substrate and a rotary sealer capable of covering the laminate by a longitudinal covering material and sealing (for example, heat seal, compression seal, and heat compression seal) a desired sectioned part and the substrate together with the surroundings of the covering material, the surroundings of the heat generating composition molded body and a necessary place of the sectioned part are heat sealed, thereby achieving a sealing treatment.

Furthermore, a magnet may be used for molding the heat generating composition of the invention. By using a magnet, it becomes possible to easily achieve accommodation of the heat generating composition in a mold and separation of the molded body from the mold, thereby making it easier to mold a heat generating composition molded body.

The "cast molding method" as referred to herein means a molding method for laminating a heat generating composition molded body on a longitudinal substance by filling in a casting mold having a concave and transferring into a substrate. In the continuous case, there is enumerated a continuous formation method in which by using a molding machine for laminating a heat generating molding molded body on a longitudinal substrate by filling in a concave and transferring into a substrate by a drum-type rotary body and a rotary sealer capable of covering the laminate by a longitudinal covering material and sealing (for example, heat seal, compression seal, and heat compression seal) a desired sectioned part and the substrate together with the surroundings of the covering material, the surroundings of the heat generating composition molded body and a necessary place of the sectioned part are heat sealed, thereby achieving a sealing treatment.

Incidentally, the heat cloth may be produced by providing an air-permeable adhesive layer at least between the heat generating composition molded body and the covering material or providing an underlay material such as non-woven fabrics between the heat generating composition molded body and the covering material. In the case of providing an air-permeable adhesive layer at least between the heat generating composition molded body and the covering material, there is no limitation so far as an air-permeable adhesive layer is present at least between the heat generating composition molded body and the covering material. For example, the air-permeable adhesive layer may be provided on the surface of the covering material opposing to the heat generating composition molded body; and the air-permeable adhesive layer may be provided on the heat generating composition molded body or a laminate of the heat generating composition molded body and the substrate and temporarily adhered under pressure or the like between the covering material and the heat generating composition molded body and/or the substrate.

Furthermore, it becomes possible to realize a high-speed production process of a heat cloth by temporarily adhering the substrate and between the heat generating composition molded body as laminated on the substrate and the covering material by an adhesive layer and then heat sealing the periphery of the heat generating composition molded body and a circumferential seal part which is the surrounding part of the heat cloth.

The heat cloth as produced by the foregoing production process can produce a flexible heat cloth having a low bending resistance and a heat cloth having high flexibility and strong nerve such that the bending resistance is low in one direction and high in the other direction.

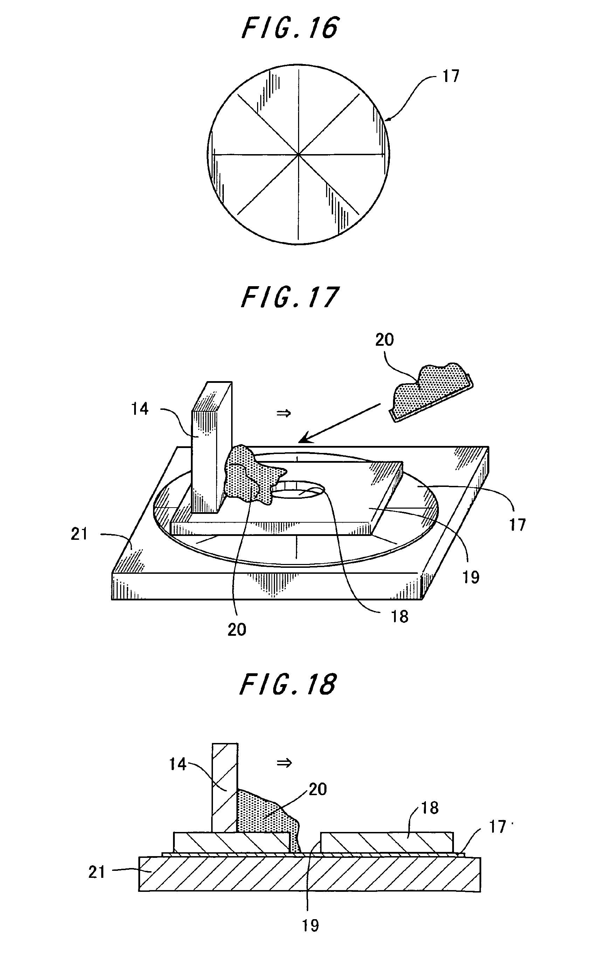

The "water mobility value" as referred to herein is a value showing an amount of surplus water which can transfer to the outside of the heat generating composition in water present in the heat generating composition. This water mobility value will be described below with reference to Figs. 16 to 20.

As shown in Fig. 16, a filter paper 17 of No. 2 (second class of JIS P3801) in which eight lines are drawn radiating from the central point with an interval of 45° is placed on a stainless steel plate 21 as shown in Figs. 17 and 18; a template 18 having a size of 150 mm in length $\times$ 100 mm in width and having a hollow cylindrical hole 19 having a size of 20 mm in inner diameter $\times$ 8 mm in height is placed in the center of the filter paper 17; a sample 20 is placed in the vicinity of the hollow cylindrical hole 19; and a stuffer plate 14 is moved on and along the template 18 and inserted into the hollow cylindrical hole 19 while stuffing the sample 20, thereby leveling the sample (force-in die molding).

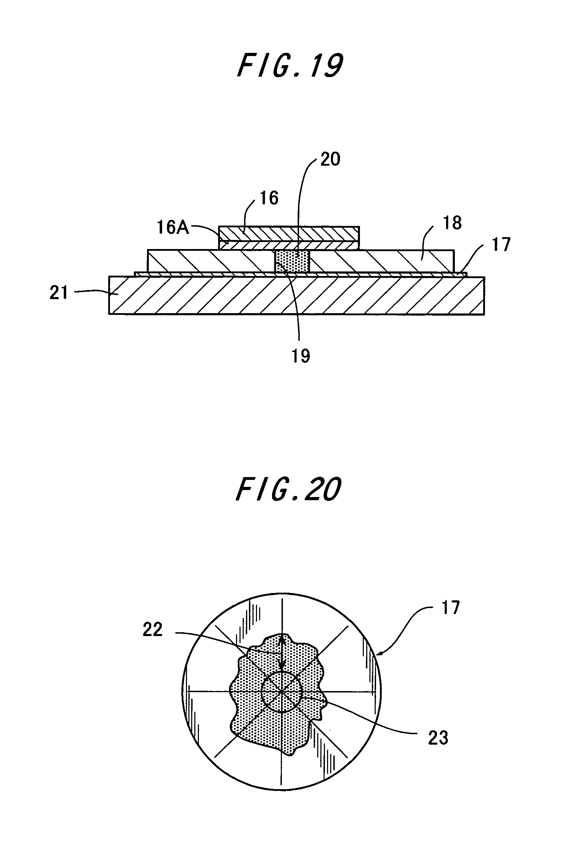

Next, as shown in Fig. 19, a non-water absorptive 70 $\mu$m-thick polyethylene film 16A is placed so as to cover the hole 19, and a flat plate 16 made of stainless steel having a size of 5 mm in thickness $\times$ 150 mm in length $\times$ 150 mm in width is further placed thereon and held for 5 minutes such that an exothermic reaction is not caused.

Thereafter, a shown in Fig. 20, the filter paper 17 is taken out, and an oozed-out locus of the water or aqueous solution is read as a distance 22 (unit: mm) from a periphery 23 as an edge of the hollow cylindrical hole to an oozed-out tip along the radiating lines. Similarly, a distance 22 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter $\times$ 8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

$$\text{(Water mobility value)} = \{[\text{Water content value (mm)}]/$$

$$[(\text{Real water content value (mm)})] \times 100$$

With respect to the same sample, five points are measured, and the five water mobility values are averaged, thereby defining an average value thereof as a water mobility value of the sample.

Furthermore, in the case of measuring the water mobility value of the heat generating composition in the heat generating body, with respect to the water content for measuring a real water content, a percentage of water content of the heat generating composition is calculated through measurement of the water content of the heat generating composition by an infrared moisture meter, a water content necessary for the measurement is calculated on the basis of the percentage of water content, and a real water content value is measured and calculated from the foregoing water content.

In the invention, a heat generating body can be formed only by laminating a heat generating composition molded body obtained by molding a heat generating composition having surplus water with a water mobility value of from 0.01 to 20 on a substrate, covering a covering material thereon, and sealing at least the periphery of the heat generating composition molded body. After accommodating it in a packaging material such as a substrate and a covering material, it is not necessary to add water. Accordingly, since the process is remarkably simplified, the invention is superior in view of the costs.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

In a heat generating body using a heat generating composition molded body obtained by molding a moldable heat generating composition containing surplus water as a connecting substance according to the invention, the heat generating composition contains an appropriate amount of surplus water expressed by a water mobility value of from 0.01 to 20 as the connecting substance without using a flocculant aid, a dry binding agent, a flocculating agent, etc.

It is assumed that when the amount of surplus water in the heat generating composition is appropriate, the surplus water causes hydration against hydrophilic groups in the components of the composition due to a bipolar mutual action or hydrogen bond, etc. and that it is present even in the surroundings of hydrophobic groups while having high structural properties. Thus, it is assumed that the heat generating composition becomes in a state of a mud ball, thereby revealing moldability. This is connecting water as a connecting substance in some meaning. Besides, there is water in a state called as free water which can freely move, and it is thought that when the surplus water increases, the structure is softened, whereby the free water increases. Furthermore, controlling factors which an iron powder causes an oxidation reaction are an amount of existing water and a feed amount of oxygen to the surface of the iron powder. It is said that in a degree of water adsorbing film (less than 100 angstroms), the water is not sufficient and that the oxidation rate is small. When the adsorbing film becomes about 1 $\mu$m, the water content becomes sufficient. Furthermore, since the thickness of the water film is thin, feed of oxygen onto the surface of the iron powder becomes easy, whereby the oxidation rate becomes large. It is assumed that when the film becomes thicker to an extent that the adsorbing film exceeds 1 $\mu$m, the feed amount of oxygen is reduced. The present inventors have obtained knowledge that the water mobility value expressing the optimal water content at which moldability and oxidation rate in fixed levels or more are revealed is from 0.01 to 20, leading to accomplishment of the invention.

That is, by using an appropriate amount of surplus water, the respective component particles are coupled with each other by a surface tension of water, moldability is generated in the heat generating composition, and the water does not substantially function as a barrier layer. Thus, the heat generating composition comes into contact with air to generate heat. In addition, by using a heat generating composition using an active iron powder or an active heat generating composition using an active iron powder, the heat generating composition becomes a heat generating composition having remarkably excellent exothermic rising properties and high moldability. Furthermore, heat generation occurs without causing transfer of the water in the heat generating composition molded body as produced by a molding and laminating system into a packaging material or water absorptive sheet. In addition, by providing plural sectional exothermic parts of the heat generating composition molded body as sectioned by seal parts, it is possible to provide a heat generating body which has flexibility itself, is excellent in installation in places where flexibility is required, such as various places of a human body and curved bodies, and is excellent in feeling for use.

Furthermore, in the substrate, the covering material and the heat generating composition molded body, by temporarily adhering at least the covering material and the heat generating composition molded body to each other via a sticky layer and then heat sealing the periphery of the heat generating composition molded body and the surroundings of the heat generating body, certainty of heat seal is improved so that it becomes possible to design to make the production speed of a heat generating body high and make the heat seal width small.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:

With respect to the measurement device, a stainless steel-made molding die (a plate having a size of 2 mm in thickness × 200 mm in length × 200 mm in width and having a cavity as treated by R5 in four corners of 60 mm in length × 40 mm in width in a central part thereof) and a fixable leveling plate are disposed above a travelable endless belt, and magnets (two magnets having a size of 12.5 mm in thickness × 24 mm in length × 24 mm in width are disposed in parallel) are disposed under the endless belt.
The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness × 200 mm in length × 200 mm in width is placed on the endless belt of the measurement device, a polyethylene film having a size of 70 μm in thickness× 200 mm in length × 200 mm in width is placed thereon, and a stainless steel-made molding die is further placed thereon.
Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 μm is not present and the number of collapsed pieces of the heat generating composition molded body having a maximum length of from 300 to 800 μm is not more than 5, it is to be noted that the heat generating composition has moldability.
The moldability is an essential property for a heat generating composition to be used in the molding system. If the heat generating composition does not have moldability, it is impossible to produce a heat generating body by the molding system.

The "perforation" as referred to in the invention includes one which is intermittently cut for the purpose of improving flexural properties of the sectioned part and one which is intermittently cut such that cutting by hand is possible. Its degree, length and aperture are not limited but are determined depending upon the desire. The perforation may be provided in all sectioned parts or may be partially provided. The shape is not particularly limited, and examples thereof include a circle, an ellipse, a rectangle, a square, and a cut line (linear shape). For example, in the perforation which is intermittently cut such that cutting by hand is possible, a circular hole having an aperture of from φ10 to 1,200 μm can be enumerated. The aperture of the hole is more preferably from φ20 to 500 μm.
It is preferable that the holes are positioned lined up in the length and width. Furthermore, a shortest space between outer peripheries of the adjacent holes in the length and width is not limited so far as it is satisfactory with flexural properties and possibility of cutting by hand. The shortest space is preferably from 10 to 2,000 μm, more preferably from 10 to 1,500 μm, further preferably from 20 to 1,000 μm, still further preferably from 20 to 500 μm, and even further preferably from 20 to 200 μm. The cutting properties by hand are remarkably improved by a balance between the aperture of the hole and the shortest space of outer peripheries of the adjacent holes in the length and width.
The hole may be a cut line, and its length may be a length corresponding to the aperture or may be larger than the aperture. A shortest space between ends of the adjacent cut lines in the length and width is corresponding to the shortest space between outer peripheries of the adjacent holes.

For example, an aperture of the hole of from $\phi 10$ to 2, 000 $\mu$m is corresponding to a length of from 10 to 2,000 $\mu$m, and a shortest space between outer peripheries of the adjacent holes in the length and width of from 10 to 2,000 $\mu$m is corresponding to a shortest space between ends of the adjacent cut lines in the length and width of from 10 to 2,000 $\mu$m. In the case of a cut line, since it becomes long in one direction, its length can be prolonged and may be from 10 to 50, 000 $\mu$m. A shortest distance between the adjacent cut lines in the length and width may be from 1 to 5,000 $\mu$m.

The "bending resistance" as referred to in the invention exhibits rigidity (tension or nerve) or flexibility and follows the A method according to JIS L1096 (45° cantilever method), except for using a heat generating body itself as a sample. That is, a heat generating body is placed on a horizontal table having a smooth surface and having a slope at an angle of 45° in one end thereof such that one side thereof coincides with a scale base line. Next, the heat generating body is slowly slid toward the slope by an appropriate method, and when a central point of the one end of the heat generating body comes into contact with the slope A, the position of the other end is read by a scale. The bending resistance is exhibited by a length (mm) for which the heat generating body moves. Respective five sheets of heat generating body are measured, and the bending resistance (calculated down to the integral place) is expressed by an average value of lengths measured in the length direction and the width direction, or in one direction and the orthogonal direction thereto. However, in the measurement, in the case of measuring an adhesive layer-provided heat generating body such that the adhesive side is faced at the horizontal table side, while the adhesive side provided with a separator is faced at the horizontal table side. In any way, a measured value in the side at which a minimum bending resistance is measured is employed.

Furthermore, in the measurement, the following must be taken into consideration.

(1) A heat generating composition-incorporated exothermic part of the heat generating body is to retain on the horizontal table to an extent of 5 mm or more in width $\times$ 20 mm or more in length. However, the length is to cross a region where the heat generating composition is present or to cross linearly a region where the heat generating composition is present and a region where the heat generating composition is not present.

(2) In the case of an adhesive layer-provided heat generating body, a plastic film having a bending resistance of not more than 30 mm, or a limp and soft film such as a limp film having a thickness of not more than 50 $\mu$m, and preferably not more than 25 $\mu$m and a plastic film in which wrinkles are formed by lightly crumpling is to be used as a separator of the adhesive layer and provided along the adhesive layer. Furthermore, with respect to the bending resistance of the substrate and/or the covering material, a specimen of 100 mm $\times$ 200 mm is prepared, and a bending resistance in the 200 mm direction is employed.

In the invention, the bending resistance in at least one direction is usually not more than 100 mm, preferably not more than 80 mm, more preferably not more than 50 mm, further preferably not more than 30 mm, and still further preferably not more than 20 mm.

A rate of bending resistance of the heat generating body or exothermic part in the invention is a rate of bending resistance to the full length of the heat generating body or exothermic part in one direction and is calculated according to the following expression.

$$\text{(Rate of bending resistance)} = (A/B) \times 100$$

Wherein A represents a bending resistance of the heat generating body or exothermic part in one direction; and B represents the full length of the heat generating body or exothermic part in the foregoing one direction.

In the invention, a rate of bending resistance in at least one direction is usually not more than 50, preferably not more than 40, and more preferably not more than 30.

A ratio of bending resistance in the invention is a ratio of a bending resistance in one direction to a smaller bending resistance in bending resistances in the directions orthogonal thereto in the plane orthogonal to the thickness direction of the heat generating body or exothermic part. The ratio of bending resistance is preferably 2 or more.

In the invention, in the case of a heat generating body having sectional exothermic parts provided at intervals in the striped form, a heat generating body provided with sectional exothermic parts of a parallelepiped shape at intervals in the striped form in which a maximum absolute value of a difference between bending resistances in the two directions as intersecting directions, a heat generating body further provided with an adhesive layer, and a heat generating body provided with adhesive layers at intervals in the striped form are very flexible in one direction and rigid in one direction. Thus, these heat generating bodies relieve symptoms such as stiff shoulders, lower-back pain, and muscular fatigue and especially exhibit efficacy for relieving a symptom of menstrual pain. In addition, these heat generating bodies are able to be wound in a size substantially equal to the width dimension in the width direction of the heat generating body, become compact and are convenient for accommodation. Furthermore, in the case of a separator-provided heat generating body, by using a separator having a low bending resistance, winding is possible.

Furthermore, in the case of providing a heat generating body along the body, the body includes many two-dimensional curves, and in shoulders, legs, abdomen, waist, arms, and the like, one direction is substantially linear, and the other two directions are formed of a substantially curved surface. Accordingly, since the heat generating body of the invention which is able to form a substantially linear surface in one direction and a curved surface in the other two directions is able to form a two-dimensional curved surface, it is able to well follow the body and is optimum for warming of the body and relaxation or treatment of various symptoms.

Furthermore, in the heat generating body of the invention, by adjusting the size or space of the convex sectional exothermic part, an exothermic part which is flexible and exhibits a uniform temperature distribution or an exothermic part exhibiting a pattern-like temperature distribution is obtainable. By the pattern-like temperature distribution, it is possible to improve a meridian effect of the warming part.

In the heat cloth having sectional exothermic parts as provided at intervals in the striped form, a minimum bending resistance on the surface orthogonal to the thickness direction thereof is preferably not more than 50 mm, more preferably not more than 40 mm, further preferably not more than 30 mm, and still further preferably from 5 to 30 mm.

Such bending resistance and bending resistance ratio are kept at least between 20 and 60 °C.

The "water retention" as referred to herein is a value as measured and calculated in the following method. That is, about 1 g of a sample fiber as prepared by cutting into a length of about 5 cm and well opening is dipped in pure water, and after elapsing 20 minutes (at 20 °C), water among the fibers is removed using a centrifuge by revolution at 2,000 rpm. A weight (W1) of the thus prepared sample is measured. Next, the sample is dried in a vacuum dryer at 80 °C until it becomes constant in weight, thereby measuring a weight (W2). A water retention is calculated according to the following expression.

$$[\text{Water retention (\%)}] = [(W1 - W2)/W2] \times 100$$

In the invention, the water retention is preferably 20 % or more.

The term "substantially planar" as referred to in the invention means a planar surface not having an accommodating concave such as an accommodating pocket, an accommodating section, and an accommodating zone as provided in advance for the purpose of accommodating the heat generating composition. Accordingly, irregularities which do not intentionally accommodate the heat generating composition may be present.

The "pocket" as referred to in the invention is an accommodating pocket which is provided in advance for the purpose of accommodating the heat generating composition and is a pocket as described in JP-T-2001-507593. Since irregularities which are not used for intentionally accommodating the heat generating composition molded body are not the pocket, even when such irregularities are present on a substrate, it is to be noted that such a substrate is defined as a substantially planar substrate.

The "accommodating section" as referred to herein is an accommodating section for accommodation as provided in advance on the packaging material for the purpose of accommodating the heat generating composition and is an accommodating section as described in Japanese Patent No. 3,161,605 and JP-T-11-508314. Since irregularities which are not used for intentionally accommodating the heat generating composition molded body are not the accommodating section, even when such irregularities are present on a substrate, it is to be noted that such a substrate is defined as a substantially planar substrate.

The "accommodating zone" as referred to herein is an accommodating zone for accommodation as provided in advance on the packaging material for the purpose of accommodating the heat generating composition and is an accommodating zone as described in Japanese Patent No. 3,161, 605 and JP-T-11-508314. Since irregularities which are not used for intentionally accommodating the heat generating composition molded body are not the accommodating zone, even when such irregularities are present on a substrate, it is to be noted that such a substrate is defined as a substantially planar substrate.

The heat generating body of the invention is able to give various shapes, thicknesses and temperature zones and therefore, can be used for various utilities such as use for a joint, facial esthetic use, use for eyes, slimming use, use for heating or warming a dripping solution, use for a wet compress pack, use for a medical body warmer, use for a neck, use for a waist, use for a mask, use for a glove, use for hemorrhage, use for relaxation of symptoms such as shoulder pain, muscular pain, and menstrual pain, use for a cushion, use for heating or warming a human body during the operation, use for a thermal sheet, use for thermally volatilizing an aroma, use for an abdomen, insecticidal use by thermal volatilization, and use for treating cancer in addition to common warming of a human body. In addition, the heat generating body of the invention can be used for heating or warming machines, pets, etc.

For example, in the case of using for relaxation of symptoms, the heat generating body of the invention is applied directly in a necessary site of the body or indirectly via a cloth, etc. Furthermore, in the case of using for heating or warming a human body during the operation, a method for using the heat generating body of the invention includes the following methods.

(1) The heat generating body is directly applied to a body requiring heating or warming.
(2) The heat generating body is fixed on a covering, etc. and covered on the body.
(3) The heat generating body is fixed on a cushion to be placed beneath the body, etc.
(4) The heat generating body is used as a covering or a cushion which is a product having the heat generating body provided therein in advance.

Incidentally, examples of the pain of muscles or bones include acute muscle pain, acute bone pain, acute reference pain, previous muscle pain, previous bone pain, chronic reference pain, and join pain of knee, elbow, etc.
The holding time is not limited but is preferably from 20 seconds to 24 hours, more preferably from one hour to 24 hours, and further preferably from 8 hours to 24 hours.
The holding temperature is preferably from 30 to 50 °C, more preferably from 32 to 50 °C, further preferably from 32 to 43 °C, still further preferably from 32 to 41 °C, and even further preferably from 32 to 39 °C.
The invention will be specifically described below with reference to the Examples, but it should not be construed that the invention is limited thereto.

[Brief Description of the Drawings]

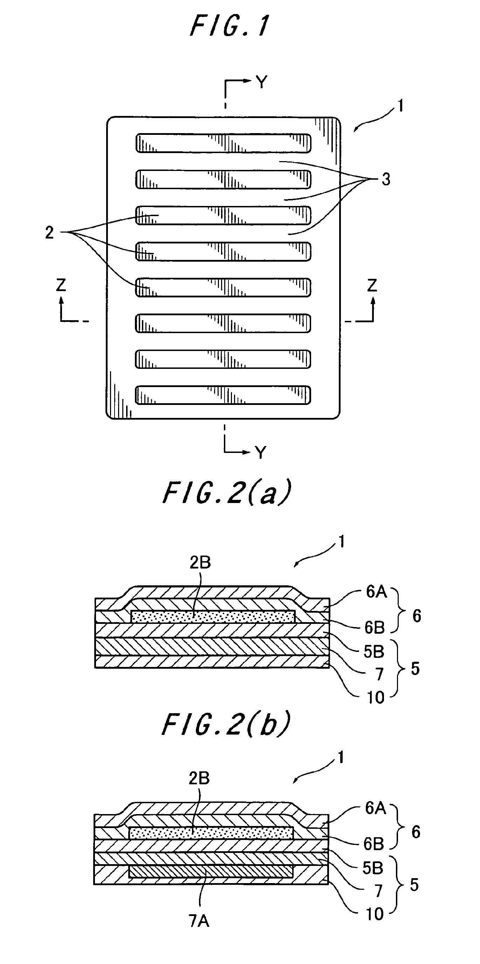

[Fig. 1] Fig. 1 is a plan view of an embodiment of the heat cloth of the invention.
[Fig. 2] Fig. 2 is a cross-sectional view along the line Z-Z of the same.

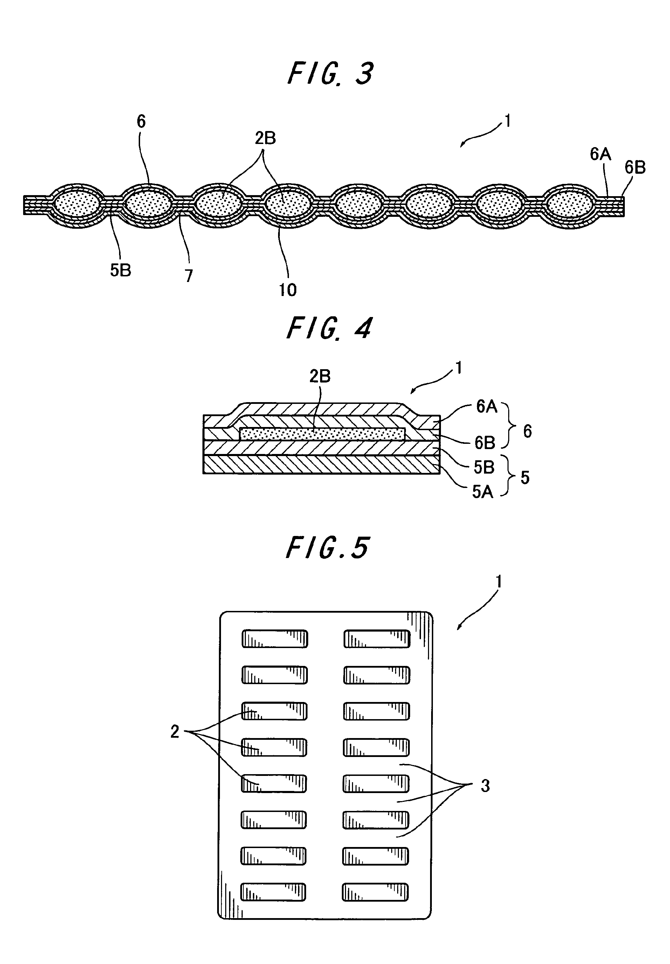

[Fig. 3] Fig. 3 is a cross-sectional view along the line Y-Y of the same.
[Fig. 4] Fig. 4 is a cross-sectional view of other embodiment of the heat cloth of the invention.
[Fig. 5] Fig. 5 is a plan view of other embodiment of the heat cloth of the invention.

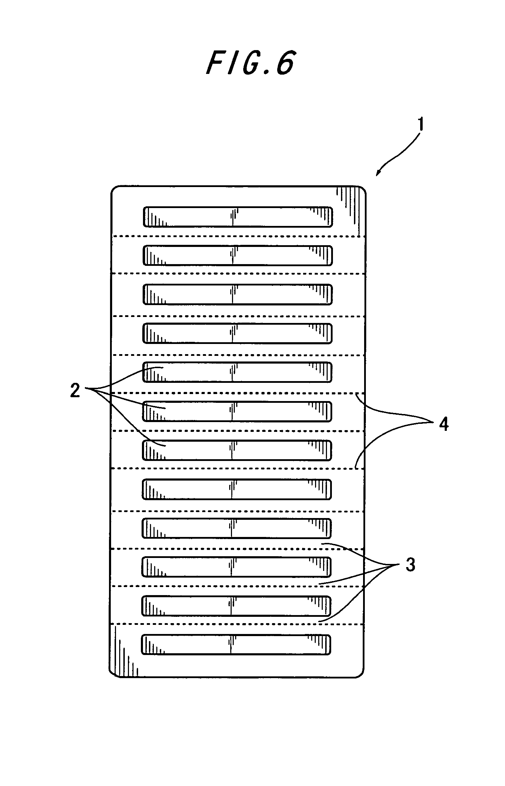

[Fig. 6] Fig. 6 is a plan view of other embodiment of the heat cloth of the invention.

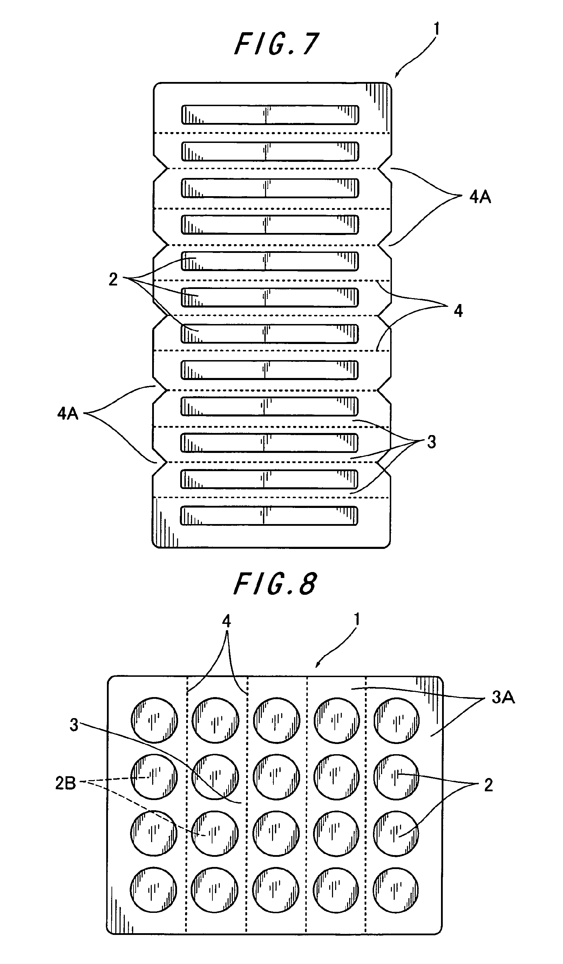

[Fig. 7] Fig. 7 is a plan view of other embodiment of the heat cloth of the invention.
[Fig. 8] Fig. 8 is a plan view of other embodiment of the heat cloth of the invention.

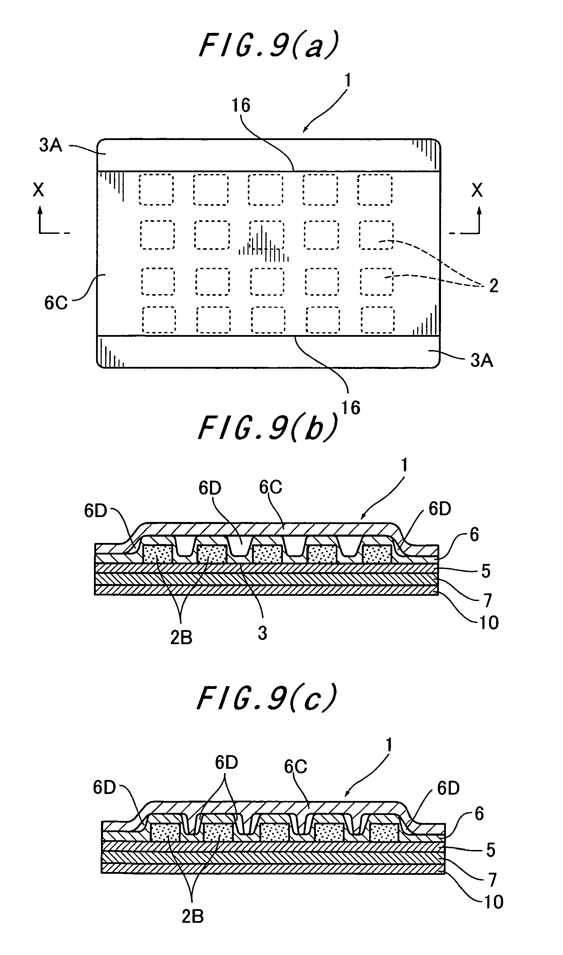

[Fig. 9] Fig. 9(a) is a plan view of other embodiment of the heat cloth of the invention; Fig. 9(b) is a cross-sectional view along the line X-X of the same; and Fig. 9 (c) is a cross-sectional view of other embodiment of the heat cloth of the invention.

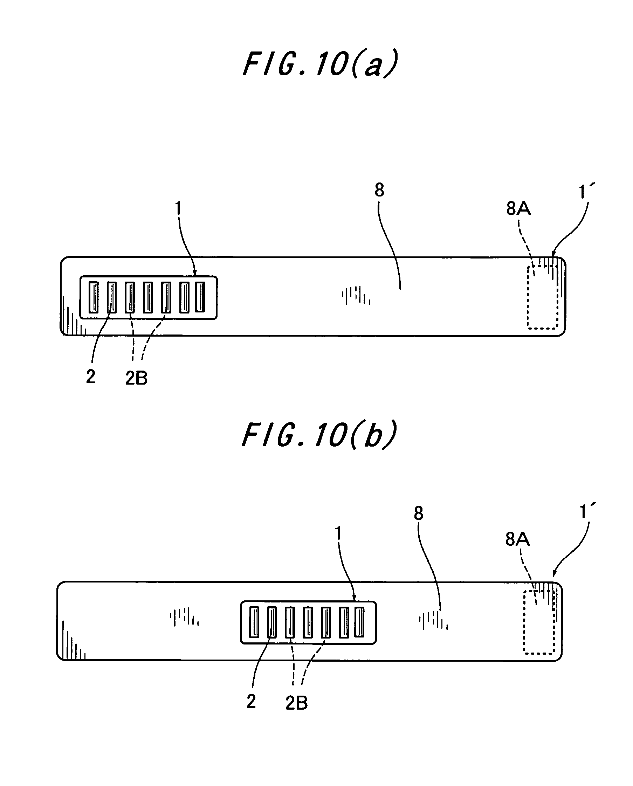

[Fig. 10] Fig. 10 is a plan view of other embodiment of the heat cloth of the invention (Fig. 10 (a) shows a heat cloth of the end part, and Fig. 10 (b) shows a heat cloth of the central part).

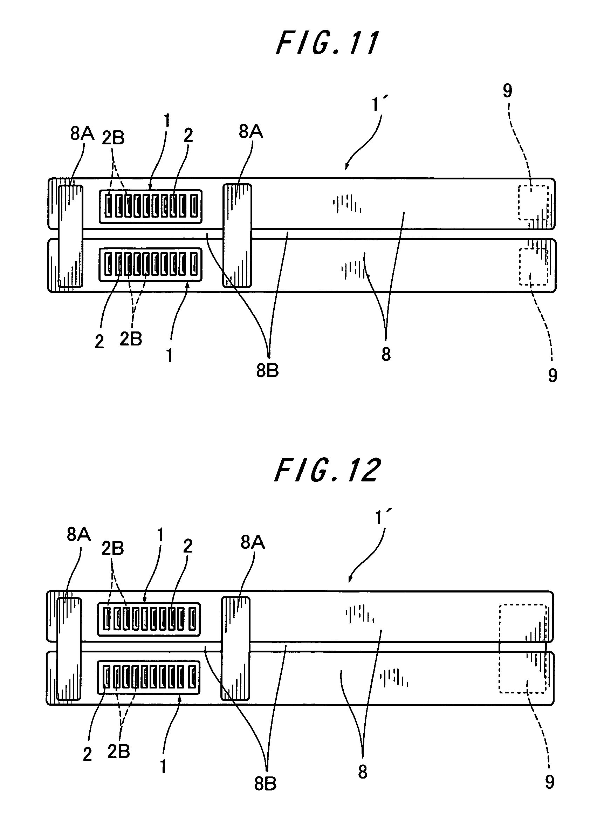

[Fig. 11] Fig. 11 is a plan view of other embodiment of the heat cloth of the invention.
[Fig. 12] Fig. 12 is a plan view of other embodiment of the heat cloth of the invention.

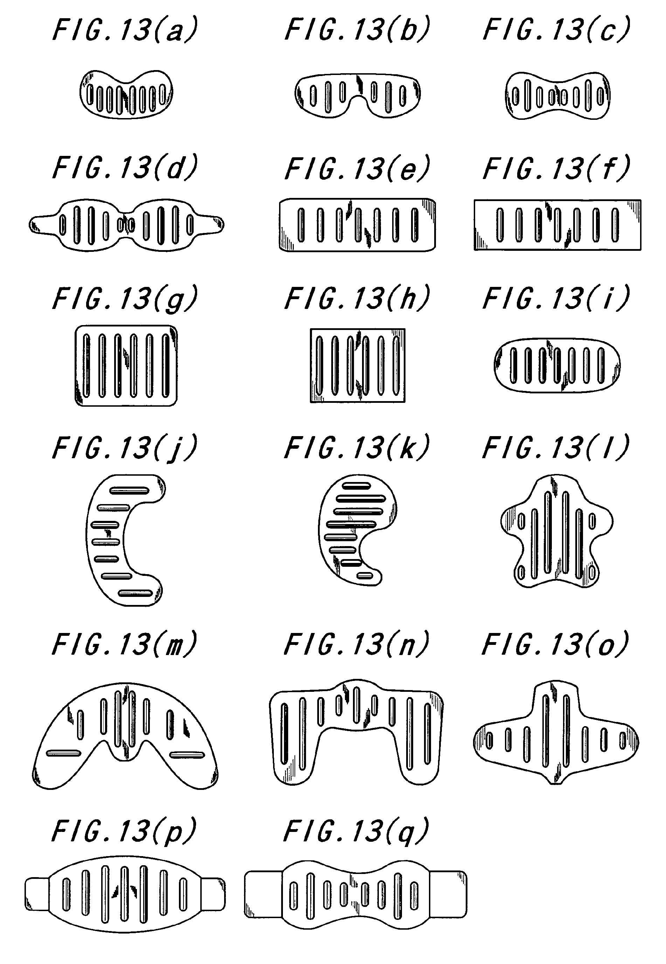

[Fig. 13] Fig. 13 is a plan view of a modified example of the shape of the heat cloth of the invention.
[Fig. 14] Fig. 14 is a schematic view of force-through molding of the heat cloth of the invention using a leveling plate.
[Fig. 15] Fig. 15 is an explanatory view in the vicinity the leveling plate of the same.
[Fig. 16] Fig. 16 is a plan view of a filter paper for the measurement of water mobility value in the invention.
[Fig. 17] Fig. 17 is an oblique view for explaining the measurement of water mobility value in the invention.
[Fig. 18] Fig. 18 is a cross-sectional view for explaining the measurement of water mobility value in the invention.
[Fig. 19] Fig. 19 is a cross-sectional view for explaining the measurement of water mobility value in the invention.
[Fig. 20] Fig. 20 is a plan view of a filter paper after carrying out the measurement of water mobility value in the invention.

[Description of Reference Numerals and Signs]

1:       Heat cloth
2:       Sectional exothermic part
2B:      Heat generating composition molded body
3:       Sectioned part
3A:      Circumferential seal part

4:        Perforation
4A:       Notch
5:        Substrate
6:        Covering material
7:        Adhesive layer
7A:       Thermal buffer sheet
8:        Band
8A:       Raw material which is stretchable in a direction intersecting the longitudinal direction
8B:       Space
9:        Fixing measure (for example, a hook and loop fastener)
10:       Separator
11:       Die
11a:      Die hole
12:       Mold
12a:      Mold hole
13:       Magnet
14:       Pushing plate
15:       Leveling plate
16:       Flat plate
16A:      Non-absorptive film (for example, a polyethylene film)
17:       Filter paper in which eight lines are drawn radiating from the central point with an interval of 45°
18:       Die plate having a hollow cylindrical hole
19:       Hole
20:       Sample
21:       Stainless steel plate
22:       Distance to the oozed-out locus of water or aqueous solution
23:       Position corresponding to a hollow cylindrical hole on filter paper

[Examples]

(Example 1)

A heat generating composition having a water mobility value of 10, which is a mixture consisting of 100 parts by weight of a reduced iron powder (particle size: not more than 300 $\mu$m), 7.0 parts by weight of active carbon (particle size: not more than 300 $\mu$m), 5.0 parts by weight of a wood meal (particle size: not more than 300 $\mu$m), 0. 8 parts by weight of a water absorptive polymer (particle size: not more than 300 $\mu$m), 0.2 parts by weight of calcium hydroxide, 0.7 parts by weight of sodium sulfite and 11 % of salt water, was used.
Next, as shown in Fig. 1, Fig. 2(a) and Fig. 3, by using the heat generating composition, heat generating composition molded bodies 2B were provided on the surface of a polyethylene film 5B of a substrate 5 made of the polyethylene film 5B provided with a 30 $\mu$m-thick acrylic adhesive layer 7 provided with a separator 10 by force-through molding using a trimming die having eight cavities of 5 mm in width $\times$ 80 mm in length at intervals of 5 mm, so as to form eight sectional exothermic parts 2; next, an air-permeable covering material 6 made of a laminate of a nylon-made non-woven fabric 6A with a basis weight of 40 g/m$^2$ on a polyethylene-made porous film 6B was covered thereon; and the peripheries of the respective heat generating composition molded bodies 2B and the circumferential part of a heat cloth 1 were sealed.
A sectioned part 3 which is a seal part between the adjacent heat generating composition molded bodies 2B was heat sealed in a seal width of 3 mm. Furthermore, the circumferential part of the heat cloth 1 was sealed in a seal width of 8 mm, thereby obtaining a heat cloth 1 having an external dimension of 98 mm in length $\times$ 91 mm in width.
Incidentally, the air permeability of the air-permeable covering material 6 was 400 g/m$^2$/24 hr in terms of a moisture permeability by the Lyssy method. With respect to the bending resistance, the long side direction of the exothermic part (direction orthogonal to the stripe direction) exhibited a minimum bending resistance and was found to be 20 mm so that a feeling for use was very excellent. Furthermore, this heat cloth 1 is able to be wound, becomes compact and is convenient for accommodation. This heat cloth 1 was sealed and accommodated in an air-impermeable accommodating bag (hereinafter referred to as "outer bag") and allowed to stand at room temperature for 24 hours. After 24 hours, the heat cloth was taken out from the outer bag and then subjected to an exothermic test of the body. As a result, it was felt warm within 3 minutes, and the warmth was continued for 7 hours. At the same time, curved surface fitness, winding properties and usefulness were evaluated. As a result, the heat cloth was superior in all of these evaluations.
Incidentally, Fig. 2(b) is a cross-sectional view of other embodiment in which a thermal buffer sheet 7A is provided in the central part of the adhesive layer.

Fig. 4 is an example of the substrate 5 in which a polypropylene-made non-woven fabric 5A is laminated on a polyethylene film 5B.

(Comparative Example 1)

Heat generating composition molded bodies were produced in the same manner as in Example 1, except for setting the water mobility value to not more than 0.01. A heat cloth was produced in the same manner as in Example 1. However, collapsed pieces of the heat generating composition molded body were scattered in the peripheries of the heat generating composition molded bodies, and cutting in seal was caused in the seal part. Furthermore, this heat cloth was subjected to an exothermic test of the body. As a result, the temperature excessively raised or the temperature was not uniform so that it was no longer useful as a heat cloth.

(Comparative Example 2)

A circumferential part of a heat cloth was sealed in a seal width of 8 mm in the same manner as in Example 1, except that sealing between the adjacent heat generating composition molded bodies 2B was not carried out, thereby obtaining a heat cloth having the same shape as in Example 1 (91 mm in length $\times$ 96 mm in width) and having an external dimension of 134 mm in length x 96 mm in width. Furthermore, the practicality of the heat cloth was evaluated in the same manner as in Example 1. As a result, the heat cloth was deteriorated in all of curved surface fitness, winding properties and usefulness.

(Example 2)

A batchwise stirring tank composed of a mixer equipped with a rotary blade in a blade form of a ventilation fan was used as an oxidizing gas contact treatment device, and air was used as an oxidizing gas. First of all, a reaction mixture consisting of 100 parts by weight of a reduced iron powder (particle size: not more than 300 $\mu$m) and 5 parts by weight of 11 % salt water and having a water mobility value of not more than 0.01 was charged in the contact treatment device vessel. Next, the upper portion of the contact treatment device vessel was opened to air, and the reaction mixture was subjected to self heat generation with stirring in the opened state to air under circumstances at 20 °C. After about 20 seconds, at a point of time when the temperature rise of the reaction mixture reached 15 °C, the reaction mixture was sealed in an air-impermeable accommodating bag and cooled to room temperature, thereby obtaining a heat generating mixture. The heat generating mixture had a wustite content of 10 %. Next, the contact treated reaction mixture was mixed with 5.3 parts by weight of active carbon (particle size: not more than 300 $\mu$m), 5 parts by weight of a wood meal (particle size: not more than 300 $\mu$m), 1. 2 parts by weight of a water absorptive polymer (particle size: not more than 300 $\mu$m), 0.2 parts by weight of calcium hydroxide, 0.7 parts by weight of sodium sulfite and 11 % of salt water, thereby obtaining a heat generating composition having a water mobility value of 5.
Next, the heat generating composition which had been molded by a trimming die in which a space having a width of 10 mm was provided in the central part thereof and eight (sixteen in total) cavities having 5 mm in width $\times$ 35 mm in length were respectively provided at intervals of 5 mm while interposing the space was laminated on a substrate.
As the substrate, an 80 $\mu$m-thick heat seal layer-provided polyethylene film was used; and as an air-permeable covering material, an adhesive layer-provided air-permeable covering material composed of an 80 $\mu$m-thick heat seal layer-provided polyethylene-made porous film and a covering material made of a nylon non-woven fabric with a basis weight of 40 g/m$^2$ having an SIS based adhesive provided in a cobweb form by a melt blow method in the porous film side was used.
Then, as shown in Fig. 5, an adhesive layer-provided heat cloth 1 was prepared by providing an SIS based adhesive in a cobweb form on the heat cloth 1 having a longest external dimension of 223 mm in length x 95 mm in width by a melt blow method. This heat cloth 1 has sectional exothermic parts 2 of 10 mm in width $\times$ 45 mm in length $\times$ 2 mm in height, a seal between the adjacent sectional exothermic parts 2 is sealed in a width of 5 mm, and two groups each composed of eight sectional exothermic parts 2 are formed. The respective groups are provided while holding a space of 7 mm in width in the center.
After temporarily adhering the substrate, the heat generating composition molded body and the covering material, the peripheries of the heat generating composition molded bodies and the surroundings of the heat cloth were heat sealed. The temporary adhering part had a seal strength at 20 °C of 200 g/25 mm. Furthermore, the heat seal part as heat sealed after temporary adhesion had a seal strength at 60 °C of 1, 500 g/25 mm. A seal width of the sectional exothermic parts is 5 mm; and a seal width of the surroundings of the heat cloth is 10 mm in the end part in the width direction and 8 mm in other part. Incidentally, the air permeability of the air-permeable covering material was 260 g/m$^2$/24 hr in terms of a moisture permeability by the Lyssy method. There was thus obtained a separator-provided heat cloth as provided with an adhesive layer in which the surface of the air-permeable covering material was made of a non-woven fabric and the other surface which is impermeable to air was provided with an SIS based adhesive in a cobweb form by a melt blow method. This heat cloth was sealed and accommodated in an outer bag and allowed to stand at room temperature for 24 hours. After 24 hours, the heat cloth was taken out from the outer bag and then subjected to an exothermic test of the body. As a result, it was felt warm within 3 minutes, and the warmth was continued for 7 hours. At the same time, curved surface fitness, winding properties and usefulness were evaluated. As a result, the heat cloth was superior in all of these evaluations.

Incidentally, though the width between the foregoing sectional exothermic parts 2 is equal, it is also possible to make the width in the both end parts shortest by making the width between the sectional exothermic parts 2 in the central part longest.

(Example 3)

By using the same heat generating composition, substrate and covering material as in Example 1, except for changing the reduced iron powder of Example 1 to an iron powder (particle size: not more than 300 μm) containing 2 % by weight of a carbon component resulting from a coating treatment of sponge iron with active carbon, heat generating composition molded bodies were molded by force-through molding using a trimming die having twelve cavities of 5 mm in width × 80 mm in length at intervals of 7 mm, laminated on the substrate and covered by the covering material; the peripheries of the heat generating composition molded bodies 2B were sealed in a seal width of 5 mm to form a sectioned part 3; the periphery of a heat cloth 1 was sealed in a seal width of 8 mm; and a perforation 4 which can be cut by hand was provided in the sectioned part 3, thereby obtaining a heat cloth 1 provided with the perforation 4 of 153 mm in length × 98 mm in width having twelve rectangular sectional exothermic parts 2 (see Fig. 6).

This heat cloth 1 was sealed and accommodated in an air-impermeable outer bag and allowed to stand at room temperature for 24 hours. After 24 hours, the heat cloth 1 was taken out from the outer bag and then subjected to an exothermic test. As a result, the temperature reached 34 °C within 3 minutes, and exothermic duration of 34 °C or higher was long as 8 hours. Furthermore, by drawing the heat cloth 1 right and left from the end in the central part by fingers along the perforation 4, two heat cloths each having six sectional exothermic parts could be prepared. The results of the exothermic test were satisfactory results, too. Furthermore, curved surface fitness, winding properties and usefulness were evaluated by a test of the body of the heat cloth 1. As a result, the heat cloth 1 was superior in all of these evaluations before and after cutting the heat cloth 1 by fingers.

(Example 4)

A heat cloth 1 the same as in Example 3 was prepared, which is an example of a V-notch 4A for tearing provided in the both end parts of the perforation (see Fig. 7).

(Example 5)

As a heat generating composition, a mixture consisting of 100 parts by weight of an iron powder (particle size: not more than 300 μm) containing 15 % by weight of FeO and 40 parts by weight of 11 % salt water (salt: 4.4 parts by weight, water: 35.6 parts by weight) was charged in a frying pan and heated for mixing in air until the water disappeared. Next, 5. 3 parts by weight of active carbon, 5 parts by weight of a 100 mesh-passed wood meal, 1.0 part by weight of a water absorptive polymer, 0.2 parts by weight of calcium hydroxide and 0. 7 parts by weight of sodium sulfite were added, and 35. 6 parts by weight of water was further added, followed by mixing to prepare a heat generating composition having a water mobility value of 8. Next, by using heat generating composition, a heat cloth 1 having an external dimension of 96 mm × 77 mm was prepared in the same manner as in Example 1, in which heat generating composition molded bodies 2 of 10 mm in length × 10 mm in width × 2 mm in height were disposed 4 lines in length and 5 lines in width at intervals of 7 mm, a heat seal width of the sectioned part was 5 mm, and a heat seal width of the surroundings of the heat cloth was 8 mm.

This heat cloth 1 was sealed and accommodated in an outer bag and allowed to stand at room temperature for 24 hours in the same manner as in Example 1. After 24 hours, the heat cloth was taken out from the outer bag and then subjected to an exothermic test of the body. As a result, it was felt warm within 3 minutes, and the warmth was continued for 8 hours. At the same time, curved surface fitness, winding properties and usefulness were evaluated. As a result, the heat cloth was superior in all of these evaluations.

(Example 6)

A batchwise stirring tank composed of a mixer equipped with a stirring blade was used as an oxidizing gas contact treatment device, and air was used as an oxidizing gas. A reaction mixture consisting of 100 parts by weight of a reduced iron powder (particle size: not more than 300 μm), 5.3 parts by weight of active carbon (particle size: not more than 300 μm), 5 parts by weight of a wood meal (particle size: not more than 300 μm), 0.8 parts by weight of a water absorptive polymer (particle size: not more than 300 μm), 0.2 parts by weight of calcium hydroxide (particle size: not more than 300 μm), 0.7 parts by weight of sodium sulfite (particle size: not more than 300 μm) and 5 parts by weight of 11 % salt water and having a water mobility value of not more than 0.01 was charged in the device vessel. Next, in the state that the upper portion of the device vessel adjusted at 20 °C was opened to air, at a point of time when the exothermic rise of the reaction mixture reached 40 °C, 11 % salt water was added to the reaction mixture with stirring to adjust the water content, thereby obtaining a heat generating composition having a water mobility value of 8.

Next, on a substrate having a separator-provided adhesive layer as provided on a polyethylene film, heat generating composition molded bodies 2B were laminated by force-through molding such that pillar-like sectional exothermic parts 2 having a diameter of 10 mm were disposed 4 lines in length and 5 lines in width at intervals of 7 mm. Next, a covering material made of a non-woven fabric-provided polyethylene-made porous film was placed thereon such that the polyethylene film and the porous film were faced at each other.

Then, the peripheries of the respective heat generating composition molded bodies 2B were heat sealed to obtain a heat cloth 1 having an exothermic part composed of twenty sectional exothermic parts 2 as shown in Fig. 8. This heat cloth is a heat cloth 1 of 96 mm in length × 77 mm in width in which a heat seal width of a sectioned part 3 is 5 mm and a heat seal width of the surroundings of the heat cloth 1 is 8 mm.

(Example 7)

A heat cloth 1 the same as in Example 6 was prepared, except for forming square sectional exothermic parts 2 in place of the circular sectional exothermic parts 2 of Example 6.

As shown in Fig. 9(b), a spacial part 6D is present between the adjacent sectional exothermic parts 2. Next, an air permeability adjusting material 6C made of a polyethylene film of 96 in length × 61 mm in width, on the entire surface of which was provided an adhesive layer, was stuck onto the non-woven fabric of the covering material 6 while leaving a the both ends of the sectional exothermic part 2 in a width of 5 mm. A sectioned part 3 is a concave; the sectional exothermic part 2 is a convex; and the sectional exothermic parts 2 become a support for the air permeability adjusting material, thereby constituting the spacial part 6D together with the sectioned part 3. This spacial part 6D works as an air-permeable layer, and the periphery in the both end parts of the sectioned part 3 works as an air intake.

This heat cloth 1 was sealed and accommodated in an air-impermeable outer bag and allowed to stand at room temperature for 24 hours. After 24 hours, the heat cloth was taken out from the outer bag and then subjected to an exothermic test on a plate as adjusted at 30 °C. As a result, the temperature reached 35 °C within one minute, and the exothermic duration at 50 to 59 °C was long as 10 hours. Fig. 9(c) shows an example in which a part of the air permeability adjusting material 6C is fixed to the sectioned part 2 via the sticky layer and each of the sectional exothermic parts 2 has an independent spacial part 6C.

Fig. 9(a) is a plan view of an embodiment of the heat cloth in which a region including the whole of plural sectional exothermic parts 2 and the both end parts of the heat cloth 1 as well as the surroundings of the sectional exothermic part 2 as formed by sealing the surroundings of the heat generating composition molded bodies 2 is covered by the air permeability adjusting material 6C; the sectioned part 3 is a concave; the sectional exothermic part 2 is a convex; the sectional exothermic part 2 works as a support of the air permeability adjusting material 6C; a spacial air-permeable layer is made of the spacial part 6D which is constituted of the air permeability adjusting material 6C and the sectioned part 3; and an air hole 16 constituted of the both end parts of the sectioned part 3, the sectional exothermic part 2 and the air permeability adjusting material 6C works as an air intake. Fig. 9 (b) is a cross-sectional view along the line X-X of Fig, 9 (a); and Fig. 9(c) shows an example in which the air permeability adjusting material 6C is fixed in the substantially central part of the sectioned part 3 and the spacial part 6D in Fig. 9 (b) is divided into two parts by the air permeability adjusting material 6C.

(Example 8)

A hydrophilic adhesive layer-provided heat cloth having the same shape as in Example 6 was prepared in the same manner as in Example 6, except for using a heat seal layer-provided laminate (0.3 g/m$^2$/day) made of a laminate of biaxially stretched polypropylene and a silicon oxide-deposited polyester film as the substrate and changing the adhesive to a hydrophilic adhesive. This heat cloth was sealed and accommodated in an air-impermeable outer bag and allowed to stand at room temperature for 24 hours. After 24 hours, the temperature was returned to room temperature, the heat cloth was then taken out from the outer bag, and the separator was removed, followed by subjecting to an exothermic test of the body. As a result, it was felt warm within 3 minutes, and the moderate warmth was continued for 8 hours or more. Furthermore, curved surface fitness, curl properties and usefulness were evaluated. As a result, the heat cloth was superior in all of these evaluations.

Furthermore, this heat cloth was sealed and accommodated in an air-impermeable outer bag and kept at 50 °C for 10 days. As a result, exothermic characteristics of the heat cloth did not change before and after keeping.

Incidentally, the hydrophilic adhesive layer was prepared in the following manner.

A component made of 4.5 % by weight of polyacrylic acid, 1.5 % by weight of poly(sodium acrylate), 4.0 % by weight of carboxymethyl cellulose, 15.0 % by weight of glycerin, 5.0 % by weight of polypropylene glycol, 0.1 % by weight of aluminum hydroxide, 6.0 % by weight of kaolin and 62.85 % by weight of water was charged in a mixing machine and thoroughly stirred until the mixture became pasty, thereby preparing a hydrophilic adhesive. This hydrophilic adhesive was uniformly coated on a separator resulting from a silicone treatment of polyethylene terephthalate (PET) having a thickness of 40 $\mu$m. This hydrophilic adhesive layer surface was stuck onto the exposed surface of the substrate of the heat cloth.

(Example 9)

By using the heat generating composition of Example 2, a non-woven fabric resulting from embossing of a heat seal layer-provided polyethylene film was laminated on the substrate, and a napped (fluffy) non-woven fabric was further laminated thereon. By using this laminate, a non-woven fabric with a basis weight of 80 g/m$^2$ was laminated on a heat seal layer-provided perforated polyethylene film and used as a covering material. This laminate had the same air permeability as in Example 2.

A heat cloth 1 having the shape as shown in Fig. 5 was prepared in the same manner as in Example 2.

As a result of subjecting the heat cloth 1 to an exothermic test, the same results as in Example 2 were obtained.

(Example 10)

Fig. 10 is a plan view to show one example of the heat cloth of the invention. In the drawing, 2 indicates a heat cloth 1 in which seven heat generating composition molded bodies 2B are accommodated in a rectangular air-permeable bag of 5 mm in thickness × 100 mm in length × 55 mm in width as formed in a flat shape. As a non-woven fabric, an extensible and air-permeable non-woven fabric was employed so as to have high fixing properties and air permeability. This air-permeable stretchable non-woven fabric-made band 8 is formed so as to impart stretchability and air permeability by partially melting the non-woven fabric on the both surfaces of a urethane based elastomer film having fine pores formed therein in an extended state in the longitudinal direction and then releasing. This band 8 could be stretched twice into a size of 1 mm in thickness × 250 mm in length × 60 mm in width and had good fixing properties and air permeability. This band 8 had a weight of 6 g, a stress at the time of 100 % extension of 300 g/25 mm and a recovery rate at the time of 100 % extension was 95 %.

Then, the band 8 is constituted of a non-woven fabric having a female fastener function of a hook and loop fastener on the both surface thereof.

Furthermore, the heat cloth 1 is stuck via an adhesive (acrylic adhesive) in a reed screen shape in a stripe form of 55 mm in length × 5 mm in width at intervals of 10 mm in the same pattern as shown in Fig. 1.

The constitution of the heat cloth 1 will be described below in detail. As the heat generating composition, the same material as in Example 2 was used. Furthermore, as the air-permeable packaging material, a laminate made of a 70 $\mu$m-thick finely porous polyethylene film on a nylon non-woven fabric with a basis weight of 40 g/m$^2$ and having a moisture permeability by the Lyssy method of 700 g/m$^2$/24 hr was used. As the air-impermeable packaging material, a 50 $\mu$m-thick polyethylene film was used.

Seven heat generating composition molded bodies having a length of 25 mm and a width of 5 mm were laminated at intervals of 6 mm on the substrate of the air-impermeable packaging material by force-through molding. Next, the air-permeable packaging material and the air-impermeable packaging material were overlaid and laminated such that the polyethylene surface of the air-permeable packaging material and the polyethylene of the air-impermeable packaging material were faced at each other, namely, the non-woven fabric was exposed; the peripheries of the heat generating composition molded bodies were heat sealed in a width of 4 mm to form a sectioned part; the surroundings of the heat cloth as the outermost surroundings of the heat generating composition molded bodies were further heat sealed in a width of 8 mm to form a circumferential seal part. There was thus prepared a heat cloth 1 having an outer diameter length of 100 mm and a width of 55 mm.

Then, the heat cloth 1 was stuck onto the band 8 via an acrylic adhesive, thereby preparing a heat cloth 1 of a band body warmer shape. Incidentally, Fig. 10(b) shows an example in which the heat cloth 1 is provided in the central part 12.

The heat cloth of a band body warm shape was sealed in an outer bag. After lapsing 24 hours, the outer bag was broken, and the heat cloth was wound around the calf of a leg by a band, fixed and provided for usual use. As a result, in a portion coming into contact with the body, which is a lower surface of the heat cloth, since stretching and non-stretching were repeated, the stretching part rose and pressed the body, whereby the pressing state was changed from the plane to a point or a line. Thus, not only the fitness became very well, but also touch to the skin was good. It was felt warm within about 3 minutes, and the warmth was continued for 6 hours or more. During the use, even by the movement, the stretching part rose and pressed the body in a point-like or linear state. Thus, a strain caused due to the movement was absorbed by the stretchability, and the stretching part neither went away nor came out depending upon the movement of the body so that deviation or dislocation of the heat cloth 1 of a band body warmer shape did not occur. The exothermic agent in the material of the invention did not move at all within the bag, was found to cause uniform heat generation over the entire surface and was free from swelling. After the use, the heat cloth 1 could be simply and smoothly detached and caused neither a pain nor inflammation on the skin. As a result of carrying out an experiment for use while moving, the heat cloth 1 did not come down.

(Example 11)

Fig. 11 is a plan view to show another example of the band body warmer of the invention.
In the drawing, 1 indicates a heat cloth 1 having a pair of heat cloths 1, 1 formed of seven sectional exothermic parts 2 in which heat generating composition molded bodies 2B are accommodated in a rectangular air-permeable bag of 5 mm in thickness × 100 mm in length × 55 mm in width as formed in a flat shape. The pair of heat cloths 1, 1 are stuck in one end side of the surface of a stretchable non-woven fabric-made band of 1 mm in thickness × 400 mm in length × 60 mm in width while interposing a space 8B via an acrylic adhesive layer which is a non-stretchable region. Furthermore, stretchable substrates 8A, 8A are provided outwardly in the both sides of the pair of heat cloths 1, 1.
This air-permeable stretchable non-woven fabric-made band 8 having a female fastener function of a hook and loop fastener is one obtained by heat melting a polyester-made non-woven fabric made of continuous filaments having a diameter of 15 $\mu$m, which is stretchable in the longitudinal direction, on the both surfaces of a stretchable urethane based elastomer film and subsequently heat embossing, thereby not only strengthening the melt but also forming fine pores. Furthermore, as an air-permeable sheet in the surface side of the heat cloths 1, 1, a non-woven fabric is used, too, on which a female fastener of a hook and loop fastener is formed. Incidentally, in the drawing, 9 indicates a male fastener.
Incidentally, the constitution of the heat cloth 1 having seven sectional exothermic parts 2 will be described below in detail. As the heat generating composition, the heat generating composition of Example 1 was used. Furthermore, as the air-permeable packaging material, a packaging material having a moisture permeability by the Lyssy method of 700 g/m$^2$/24 hr, in which a 70 $\mu$m-thick polyethylene-made porous film was laminated on a nylon non-woven fabric with a basis weight of 40 g/m$^2$ and having a female fastener function of a hook and loop fastener, was used. As the air-impermeable packaging material, a 50 $\mu$m-thick polyethylene film was used. Ten heat generating composition molded bodies of a rectangle of 7 mm in width × 25 mm in length × 2 mm in height were provided in intervals of 5 mm on the polyethylene film of the air-impermeable packaging material by cast molding using a cast die in which ten rectangles of 7 mm in width × 25 mm in length × 2 mm in height were provided at intervals of 5 mm. Next, the air-permeable packaging material and the air-impermeable packaging material were overlaid and laminated such that the porous film surface of the air-permeable packaging material and the polyethylene film surface of the air-impermeable packaging material were faced at each other, namely, the non-woven fabric was exposed; and the surroundings of the heat generating composition molded bodies were heat sealed in a seal width of 3 mm, and the surroundings of the heat cloth 1 were heat sealed in a seal width of 8 mm, thereby producing the heat cloth 1.
The heat cloth 1' of a band body warmer shape was sealed in an outer bag and after lapsing 24 hours, was taken out from the outer bag. A kneecap was put into a space 8B of the band body warmer, wound by a band, fixed and then provided for usual use. As a result, it was felt warm within about 3 minutes, and the warmth was continued for 6 hours or more. The exothermic agent in the material of the invention did not move at all within the bag and was found to cause uniform heat generation over the entire surface. In long-term use, it was felt comfortable, and after the use, the entire heat cloth 1' could be simply and smoothly detached and caused neither a pain nor inflammation on the skin.
Fig. 12 is an example in which a male fastener 9 of a hook and loop fastener as the fixing measure of Fig. 11 is provided crossing bands 8, 8 such that one end side of the bands 8, 8 is not separated away.

(Example 12)

A heat generating mixture consisting of 100 parts by weight of an iron powder having a wustite content of less than 1 % (particle size: not more than 300 $\mu$m), 2.5 parts by weight of active carbon (particle size: not more than 300 $\mu$m), 3 parts by weight of a water absorptive polymer (particle size: not more than 300 $\mu$m), 0.5 parts by weight of calcium hydroxide, 0.7 parts by weight of sodium sulfite and 5 parts by weight of 11 % salt water and having a water mobility value of not more than 0.01 was charged in a contact treatment device vessel. Next, the upper portion of the contact treatment device vessel was opened to air, and the reaction mixture was subjected to a self heat generation with stirring in the opened state to air under circumferences at 20 °C. At a point of time when the temperature rise of the reaction mixture reached 10 °C, 11 % salt water was mixed to obtain a heat generating composition having a water mobility value of 10. Next, by using the heat generating composition and using the same substrate and covering material and the same manner as in Example 1, twenty heat generating molded bodies constituting a sectional exothermic part were provided on the surface of a polyethylene film of a substrate made of a laminate of a nylon-made non-woven fabric and a polyethylene film by force-through molding using a trimming die having twenty cavities of 5 mm in width × 80 mm in length at intervals of 5 mm. Next, an air-permeable covering material made of a laminate of a polyethylene-made porous film and a nylon-made non-woven fabric was covered thereon, and the peripheries of the respective heat generating composition molded bodies and the surroundings of the heat cloth were sealed. The surroundings of the respective sectional exothermic parts were heat sealed in a seal width of 3 mm, the surroundings of the heat cloth were sealed in a seal width of 8 mm, and the central exothermic parts were sealed in a width of 8 mm, thereby obtaining a heat cloth having an external dimension of 211 mm in length × 96 mm in width. This heat cloth was sealed and accommodated in an outer bag and allowed to stand at room temperature for 24 hours. After 24 hours, the heat cloth was taken out from the outer bag and then subjected to an exothermic test of the body. As a result, it was felt warm within 3 minutes, and the warmth was continued for 8 hours. At the same time, curved surface fitness, winding properties and usefulness were evaluated. As a result, the heat cloth was superior in all of these evaluations. Incidentally, the external dimension is not particularly limited, and for example, one having an external dimension of 1 m and a width of 96 mm can be formed.

(Example 13)

As a heat generating composition, a heat generating composition having a water mobility value of 8, which is a mixture consisting of 100 parts by weight of a reduced iron powder (particle size: not more than 300 $\mu$m), 7.0 parts by weight of active carbon (particle size: not more than 300 $\mu$m), 5.0 parts by weight of a wood meal (particle size: not more than 300 $\mu$m), 0. 8 parts by weight of a water absorptive polymer (particle size: not more than 300 $\mu$m), 0.2 parts by weight of calcium hydroxide, 0.7 parts by weight of sodium sulfite and 11 % of salt water, was used. Next, by using the heat generating composition, five heat generating molded bodies constituting a sectional exothermic part were provided on the surface of a polyethylene film of a substrate made of a 30 $\mu$m-thick acrylic adhesive layer-provided polyethylene film as provided with a separator by force-through molding using a trimming die having five cavities of 5 mm in width × 80 mm in length at intervals of 5 mm.
Next, an air-permeable covering material made of a laminate of a polyethylene-made porous film on a nylon-made non-woven fabric with a basis weight of 40 g/m$^2$ was passed through a folding machine composed of a pair of folding tools having a concavo-convex surface and folded in a wavy shape. Then, valleys of the covering material were pressed onto the substrate by using one of the folding tools having a concavo-convex surface; the heat generating composition molded bodies constituting a sectional exothermic part were wrapped and covered within crests of the covering material; and the covering material and the substrate in a corresponding region of the sectioned part were heat sealed. Next, the peripheries of the heat generating composition molded bodies were heat sealed, and the surroundings of the heat cloth were further heat sealed, thereby obtaining a heat cloth having an exothermic part made of sectional exothermic parts in a stripe form. The sectioned part which is a seal part of the periphery of the respective heat generating composition molded body was heat sealed in a seal width of 3 mm, thereby preparing the sectional exothermic part as sectioned by the sectioned part. Furthermore, by sealing the surroundings of the heat cloth in a seal width of 8 mm, the heat cloth having a sectional exothermic part in a stripe form of 98 mm in length × 91 mm in width in terms of an external dimension was obtained. Incidentally, the air permeability of the air-permeable covering material was 400 g/m$^2$/24 hr in terms of a moisture permeability by the Lyssy method. This heat cloth was sealed and accommodated in an air-impermeable accommodating bag (hereinafter referred to as "outer bag") and allowed to stand at room temperature for 24 hours. After 24 hours, the heat cloth was taken out from the outer bag and then subjected to an exothermic test of the body. As a result, it was felt warm within 3 minutes, and the warmth was continued for 7 hours. At the same time, curved surface fitness, winding properties and usefulness were evaluated. As a result, the heat cloth was superior in all of these evaluations.

(Example 14)

Fig. 13 shows examples of the shape of the heat cloth of the invention.

(a) shows a broad bean-like shape; (b) shows an eye mask-like shape; (c) shows a cocoon-like shape; (d) shows a gourd-like shape; (e) shows a rectangular shape with rounded corners; (f) shows a rectangular shape; (g) shows a square shape with rounded corners; (h) shows a square shape; (i) shows an egg-like shape; (j) shows a boomerang-like shape; (k) shows a comma-shaped bead-like shape; (1) shows a wing-like shape; (m) shows a wing-like shape; (n) shows a star-like shape; (o) shows a nose-like shape; (p) shows a paper lantern-like shape; and (q) shows a paper lantern-like shape, respectively.
Furthermore, while the directions of the long axes along the long sides of the rectangles of the sectional exothermic parts are parallel to each other, they may be arbitrarily set up. Also, a gathering of sectional exothermic parts in different directions may be employed. Modified shapes as modified on the basis of these basic skeletons can also be used.

(Example 15)

Fig. 14 shows an embodiment of the force-through molding method using a leveling plate 15. That is, a substrate 3 in a roll film form having a width of 130 mm and a thickness of 1 mm is adapted to a molding mold 12 having a thickness of 1.5 mm and having a desired shape punched out in the center thereof and horizontally sent at a prescribed speed between a die 11 as disposed in the upper surface and a magnet 13 as disposed in the lower surface. The heat generating composition 2 of the invention is sent into a mold hole 12a from the upper surface of the mold 12 through a hole 11a of the die 11. The heat generating composition 2' is leveled in the same level as in the mold 12 by a leveling plate 15 as placed forward in the advancing direction and accommodated in the mold hole 12a, whereby a shape having a thickness of 1.5 mm is molded on the substrate 3. Thereafter, the mold 12 is removed to obtain a heat generating composition molded body as laminated on the substrate 5. While not illustrated, a styrene-isoprene-styrene block copolymer (SIS) based sticky polymer is then provided in a netlike form on the surface of the foregoing molded body by the melt blow method, a covering material is covered thereon, and the periphery of the molded body is sealed by heat seal, followed by cutting into a desired shape. There is thus obtained a heat cloth having a desired shape. In addition, the cut heat cloth of the invention is subsequently sent into a packaging step and sealed in an air-tight outer bag. Furthermore, the same molding is possible even by changing the leveling plate to a pushing leveling plate. Fig. 15 shows an enlarged view of the leveling plate 15.

**Claims**

1. A heat cloth having a heat generating composition molded body resulting from molding a heat generating composition containing surplus water as a connecting substance accommodated in an accommodating bag, wherein the accommodating bag is constituted of a substrate and a covering material; the covering material covers the heat generating composition molded body as provided on the substrate; the periphery of the heat generating composition molded body is heat sealed to form irregularities; sectional exothermic parts of a convex in which the heat generating composition molded body is accommodated are disposed while holding a sectioned part of a concave as a heat seal part; and the exothermic part is formed of a gathering of the sectional exothermic parts,
   **characterized in that**:

   1) the substrate is substantially planar and does not contain a pocket, an accommodating division or an accommodating section,
   2) the heat generating composition contains, as essential components, an iron powder, a carbon component, a reaction accelerator and water, does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air,
   3) a volume of the heat generating composition molded body is from 0.1 to 30 cm$^3$,
   4) a ratio of the capacity of the sectional exothermic parts to the volume of the heat generating composition molded body is from 0.6 to 1.0,
   5) a maximum height of the sectional exothermic parts is from 0.1 to 10 mm,
   6) a width of the sectioned part as a space between the sectional exothermic parts is from 0.3 to 50 mm,
   7) a minimum bending resistance on the surface orthogonal to at least a thickness of the heat cloth is not more than 100 mm,
   8) a part of the accommodating bag has air permeability, and
   9) the surroundings of the accommodating bag are sealed.

2. The heat cloth according to claim 1, **characterized in that** the heat generating composition contains a component resulting from a contact treatment of a mixture containing at least an iron powder, a carbon component, a reaction accelerator and water as essential components with an oxidizing gas.

3. The heat cloth according to claim 1, **characterized in that** the iron powder comprising particles, a surface of each of which is at least parially covered with an iron oxide film, the iron oxide film has a thickness of 3 nm or more, and the iron powder at least contains from 20 to 100 % by weight of an active iron powder particles having a region of an oxygen-free iron component in at least one region selected from a central part region of the iron powder particles and a region beneath the iron oxide film.

4. The heat cloth according to claim 1, **characterized in that** the iron powder is covered on at least a part of the surface thereof by a wustite film and contains from 20 to 100 % by weight of an active iron powder having an amount of wustite of from 2 to 50 % by weight in terms of an X-ray peak intensity ratio to iron.

5. The heat cloth according to claim 1, **characterized in that** the heat generating composition molded body is compressed.

6. The heat cloth according to claim 1, **characterized in that** in the heat seal part, the heat seal part is formed by heat sealing after temporary adhesion by an adhesive layer, and an adhesive component which constitutes the adhesive layer and a heat seal material component which constitutes the heat seal part are copresent in at least a part of the heat seal part.

7. The heat cloth according to claim 1, **characterized in that** after heat sealing, at least a part of the heat generating composition molded body is moved to a temporary adhering part which is not heat sealed, thereby deadhering the temporary adhering part which is not heat sealed.

8. The heat cloth according to claim 1, **characterized in that** at least a part of the air-permeable surface of the exothermic part in which the sectional exothermic parts are continuously provided is covered by an air permeability adjusting material.

9. The heat cloth according to claim 1, **characterized in that** the sectioned part is provided with a perforation.

10. The heat cloth according to claim 1, **characterized in that** at least a part of the sectioned part has an irregular pattern.

11. The heat cloth according to claim 1, **characterized in that** the accommodating bag has a fixing measure on at least a part of the exposed surface thereof.

12. The heat cloth according to claim 11, **characterized in that** the fixing measure is an adhesive layer and optionally is provided with a separator.

13. The heat cloth according to claim 12, **characterized in that** the adhesive layer is a hydrophilic adhesive layer, and a packaging material between the hydrophilic adhesive layer and the heat generating composition molded body has a moisture permeability of not more than 2 $g/m^2/24$ hr.

14. The heat cloth according to claim 12, **characterized in that** the adhesive layer has air permeability.

15. The heat cloth according to claim 12, **characterized in that** the fixing measure is a sheet-like material in which a non-stretchable portion and a stretchable portion are integrally formed in a sheet-like form, and the heat cloth is provided in the non-stretchable portion of the sheet-like material.

16. The heat cloth according to claim 1, **characterized in that** the minimum bending resistance on the surface orthogonal to the thickness direction of the heat cloth is not more than 100 mm.

17. The heat cloth according to claim 1, **characterized in that** the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

18. A process for producing a heat cloth having a heat generating composition molded body accommodated in an air-permeable accommodating bag, **characterized in that**:

1) the accommodating bag is constituted of a substrate and a covering material; the heat generating composition molded body is formed by molding a heat generating composition containing surplus water as a connecting

**45**

substance; the covering material covers the heat generating composition molded body as provided on the substrate; the periphery of the heat generating composition molded body is heat sealed to form irregularities; sectional exothermic parts of a convex in which the heat generating composition molded body is accommodated are disposed while holding a sectioned part of a concave as a heat seal part; and the exothermic part is formed of a gathering of the sectional exothermic parts,

2) the substrate is substantially planar and does not contain a pocket, an accommodating division or an accommodating section,

3) the heat generating composition contains, as essential components, an iron powder, a carbon component, a reaction accelerator and water, with an amount of water contained in the heat generating composition being from 1 to 60 % by weight, does not contain a flocculant aid, a flocculant, an agglomeration aid, a dry binding material, a dry binding agent, a dry binder, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air,

4) a volume of the heat generating composition molded body is from 0.1 to 30 cm$^3$, and a ratio of the capacity of the sectional exothermic parts to the volume of the heat generating composition molded body is from 0.6 to 1.0,

5) a maximum height of the sectional exothermic parts is from 0.1 to 10 mm,

6) a width of the sectioned part as a space between the sectional exothermic parts is from 0.3 to 50 mm,

7) a minimum bending resistance on the surface orthogonal to at least a thickness of the heat cloth is not more than 100 mm,

8) a part of the accommodating bag has air permeability, and

9) the surroundings of the accommodating bag are sealed.

19. The process for producing a heat cloth according to claim 18, **characterized in that** the substrate and the covering material each have a heat seal layer; an adhesive layer made of an adhesive is provided on at least one of the heat seal layers; and the substrate and the covering material are temporarily adhered via the adhesive layer in the surrounding of the heat generating composition molded body and then heat sealed.

20. The process for producing a heat cloth according to claim 19, **characterized in that** heat sealing is carried out in a width narrower than that of the temporary adhering seal part, and thereafter, the heat generating composition is moved into a region which is not heat sealed within the temporary adhering seal part, thereby achieving deadhesion.

*FIG.1*

*FIG.2(a)*

*FIG.2(b)*

## FIG. 3

## FIG. 4

## FIG. 5

# FIG.6

## FIG.7

## FIG.8

## FIG.9(a)

## FIG.9(b)

## FIG.9(c)

# FIG.10(a)

# FIG.10(b)

# FIG.11

# FIG.12

FIG.13(a)

FIG.13(b)

FIG.13(c)

FIG.13(d)

FIG.13(e)

FIG.13(f)

FIG.13(g)

FIG.13(h)

FIG.13(i)

FIG.13(j)

FIG.13(k)

FIG.13(l)

FIG.13(m)

FIG.13(n)

FIG.13(o)

FIG.13(p)

FIG.13(q)

# FIG.14

# FIG.15

EP 1 782 777 A1

## *FIG.16*

## *FIG.17*

## *FIG.18*

56

## FIG.19

## FIG.20

## INTERNATIONAL SEARCH REPORT

| International application No. |
|---|
| PCT/JP2005/013007 |

| A. CLASSIFICATION OF SUBJECT MATTER |
|---|
| Int.Cl$^7$ A61F7/08, A61B19/08, C09K5/16 |

According to International Patent Classification (IPC) or to both national classification and IPC

| B. FIELDS SEARCHED |
|---|
| Minimum documentation searched (classification system followed by classification symbols) |
| Int.Cl$^7$ A61F7/08, A61B19/08, C09K5/16 |

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2003-334212 A (Maikoru Kabushiki Kaisha), 25 November, 2003 (25.11.03), Full text; Fig. 2 (Family: none) | 1,2,11,12 |
| A | JP 2003-336042 A (Maikoru Kabushiki Kaisha), 28 November, 2003 (28.11.03), Full text; Figs. 1 to 16 & US 2004/0217325 A    & EP 1516900 A & WO 2003/097764 A1    & CA 2439044 A & CN 1496395 A | 1,2,5,18 |
| Y | JP 2002-155273 A (Kaoru USUI), 28 May, 2002 (28.05.02), Full text; Figs. 1 to 14 (Family: none) | 1,2,5,6,13, 14,17,18,19 |

| ☒ Further documents are listed in the continuation of Box C. | ☐ See patent family annex. |
|---|---|

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 16 September, 2005 (16.09.05) | 11 October, 2005 (11.10.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

INTERNATIONAL SEARCH REPORT

International application No.

PCT/JP2005/013007

C (Continuation).  DOCUMENTS CONSIDERED TO BE RELEVANT

| Category* | Citation of document, with indication, where appropriate, of the relevant passages | Relevant to claim No. |
|---|---|---|
| Y | CD-ROM of the specification and drawings annexed to the request of Japanese Utility Model Application No. 5759/1992 (Laid-open No. 58123/1993) (Shozo NAKAJIMA), 03 August, 1993 (03.08.93), Full text; Fig. 1 (Family: none) | 1,18,19 |
| Y | JP 2003-204983 A (Asahi Kasei Corp.), 22 July, 2003 (22.07.03), Column 4, line 32 to 46; page 5, line 16 to column 6, line 40 (Family: none) | 1,16,18 |
| A | JP 11-512954 A (The Procter & Gamble Co.), 09 November, 1999 (09.11.99), Full text; Figs. 1 to 3 & JP 3545769 B          & WO 1997/049361 A1 & AU 735088 B | 8 |
| Y | JP 3007467 U (Kiribai Kagaku Kabushiki Kaisha), 14 February, 1995 (14.02.95), Page 9, lie 17 to page 10, line 14; Figs. 1 to 8 (Family: none) | 9 |
| Y | JP 2003-509120 A (The Procter & Gamble Co.), 11 March, 2003 (11.03.03), Full text; Figs. 1, 2 & US 6336935 B1          & EP 1212020 A & WO 2001/019302 A1      & AU 7116900 A & BR 14044 A              & CN 1373649 A & CA 2381812 A            & AU 768363 B | 10 |
| A | JP 10-17907 A (Dowa Iron Powder Co., Ltd.), 20 January, 1998 (20.01.98), Full text; Fig. 1 (Family: none) | 4 |
| Y | JP 2001-513394 A (The Procter & Gamble Co.), 04 September, 2001 (04.09.01), Full text; Figs. 1 to 6 & US 5904710 A1          & EP 1021145 A & WO 1999/009918 A1      & DE 69820661 T & NO 20000846 A          & BR 9811970 A & CA 2301137 A            & CN 1271265 A & HU 2689 A               & CZ 20000585 A & AU 739013 B             & AT 256446 T & DK 1021145 T            & ES 2209165 T | 15 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4293989 A
- JP 6343658 A
- JP 59189183 A
- WO 0013626 A
- JP 9075388 A
- JP 60101448 A
- JP 9276317 A
- JP 11299817 A
- JP 1110718 A
- JP 6026829 A
- JP 2000288008 A
- JP 11507593 T
- JP 11508314 T
- JP 11508786 T

- JP 11512954 T
- JP 2002514104 T
- JP 7124193 A
- JP 2002200108 A
- JP 10265373 A
- JP 9087173 A
- JP 6145050 A
- JP 6199660 A
- JP 10279466 A
- JP 10182408 A
- JP 3161605 B
- JP 2001507593 T
- JP 7194641 A
- JP 3161605 A